# EUROPEAN PATENT APPLICATION

(11) **EP 3 363 900 A1**
(43) Date of publication of application: **22.08.2018**
(21) Application number: 17157132.6
(22) Date of filing: 21.02.2017
(51) Int. Cl.: C12N 15/10

(54) **EVOLUTION-GUIDED MULTIPLEXED DNA ASSEMBLY OF DNA PARTS, PATHWAYS AND GENOMES**

(71) Applicant: ETH Zurich, 8092 Zurich (CH)
(72) Inventor: CHRISTEN, Matthias, 4153 Reinach BL (CH); CHRISTEN, Beat, 5400 Baden (CH); CHRISTEN, Heinz, 4104 Oberwil (CH)

(57) **Abstract**

The invention relates to a process for assembling DNA parts into multi-kilo base long synthetic DNA constructs. The process generates multiple, synonymous DNA parts in parallel and selects in a combinatorial assembly approach for those sequence variants with the best synthesis and assembly feasibility. DNA parts are sequence optimized and partitioned into synonymous variant designs that serve as redundant building units for higher order DNA assembly. The major stages of the process are: computational partitioning and synonymous recoding of the DNA design, DNA synthesis of sequence variants pools, serial PCR to isolate sets of DNA parts and higher order assembly. As the higher-order assembly does no longer depends on successful synthesis of each DNA part, large-scale DNA designs can be quickly completed allowing for cost-effective and highly parallelised assembly of synthetic bio-designs.

SEQUENCE LISTING

<110>  ETH Zuerich

<120>  Evolution-guided Multiplexed DNA Assembly of DNA Parts, Pathways
       and Genomes

<130>  eth161ep

<160>  50

<170>  PatentIn version 3.5

<210>  1
<211>  43
<212>  DNA
<213>  Artificial Sequence

<220>
<223>  Adapter sequence used for partitioning, 5' segment adapter

<400>  1
cggatttcaa tagctgatat agcgaatcac cgagattaat taa                        43


<210>  2
<211>  43
<212>  DNA
<213>  Artificial Sequence

<220>
<223>  Adapter sequence used for partitioning, 3' segment adapter

<400>  2
gtttaaacga tactagatgt ataatgtccg ccatgcagac gaa                        43


<210>  3
<211>  33
<212>  DNA
<213>  Artificial Sequence

<220>
<223>  Adapter sequence used for partitioning, 5' block adapter

<400>  3
cgagttttgg ggagacgacc atatggctct tca                                   33


<210>  4
<211>  33
<212>  DNA
<213>  Artificial Sequence

<220>
<223>  Adapter sequence used for partitioning, 3' block adapter

<400>  4
cgagttttgg ggagacgacc atatggctct tca                                   33


<210>  5
<211>  18

<212>  DNA
<213>  Artificial Sequence

<220>
<223>  Barcode primer used for subpool PCR amplification,
       5'-barcode1_blc_0

<400>  5
gcgttcgctc taagagtc                                                    18


<210>  6
<211>  18
<212>  DNA
<213>  Artificial Sequence

<220>
<223>  Barcode primer used for subpool PCR amplification, 5'-bar2_blc_1

<400>  6
agtcgtctca tcggtagc                                                    18


<210>  7
<211>  18
<212>  DNA
<213>  Artificial Sequence

<220>
<223>  Barcode primer used for subpool PCR amplification, 5'-bar3_blc_2

<400>  7
ggctgatact cgctacgt                                                    18


<210>  8
<211>  18
<212>  DNA
<213>  Artificial Sequence

<220>
<223>  Barcode primer used for subpool PCR amplification, 5'-bar4_blc_3

<400>  8
gccgtcggta gttcatac                                                    18


<210>  9
<211>  18
<212>  DNA
<213>  Artificial Sequence

<220>
<223>  Barcode primer used for subpool PCR amplification, 5'-bar5_blc_4

<400>  9
ctttccctag acggaggt                                                    18


<210>  10
<211>  18
<212>  DNA
<213>  Artificial Sequence

<220>
<223>  Barcode primer used for subpool PCR amplification, 3'-bar6_segm25

<400>  10
cgtccggttg aagtctac                                                    18


<210>  11
<211>  18
<212>  DNA
<213>  Artificial Sequence

<220>
<223>  Primer used for PCR verification of block assembly, Primer ID
       BC1484

<400>  11
gtgaaggtga gccagtga                                                    18


<210>  12
<211>  18
<212>  DNA
<213>  Artificial Sequence

<220>
<223>  Primer used for PCR verification of block assembly, Primer ID
       BC1485

<400>  12
gaaagtcaaa agcctccg                                                    18


<210>  13
<211>  20
<212>  DNA
<213>  Artificial Sequence

<220>
<223>  Primer used for PCR verification of block assembly, Primer ID A1

<400>  13
cctgcacagg ctcgacgatg                                                  20


<210>  14
<211>  20
<212>  DNA
<213>  Artificial Sequence

<220>
<223>  Primer used for PCR verification of block assembly, Primer ID A2

<400>  14
cgttcgccga cgtggtgttc                                                  20


<210>  15
<211>  20
<212>  DNA
<213>  Artificial Sequence

<220>
<223>  Primer used for PCR verification of block assembly, Primer ID A3

<400>  15
gccaagcaac taggcggcgt                                                   20


<210>  16
<211>  20
<212>  DNA
<213>  Artificial Sequence

<220>
<223>  Primer used for PCR verification of block assembly, Primer ID A4

<400>  16
gcgacgaccg cagaaggtga                                                   20


<210>  17
<211>  20
<212>  DNA
<213>  Artificial Sequence

<220>
<223>  Primer used for PCR verification of block assembly, Primer ID A5

<400>  17
cctgtcaggt gctggtctgg                                                   20


<210>  18
<211>  20
<212>  DNA
<213>  Artificial Sequence

<220>
<223>  Primer used for PCR verification of block assembly, Primer ID A6

<400>  18
ggcgatccga gacgaagtcg                                                   20


<210>  19
<211>  20
<212>  DNA
<213>  Artificial Sequence

<220>
<223>  Primer used for PCR verification of block assembly, Primer ID A7

<400>  19
ccacacccat catgcgcacg                                                   20


<210>  20
<211>  20
<212>  DNA
<213>  Artificial Sequence

<220>
<223>  Primer used for PCR verification of block assembly, Primer ID A8

<400>  20
tccgctggtg atcgacctgg                                                   20


<210>  21
<211>  20
<212>  DNA
<213>  Artificial Sequence

<220>
<223>  Primer used for PCR verification of block assembly, Primer ID A9

<400>  21
cgcgtgctat aggcgagcca                                                   20


<210>  22
<211>  20
<212>  DNA
<213>  Artificial Sequence

<220>
<223>  Primer used for PCR verification of block assembly, Primer ID A10

<400>  22
gcgcatcggc ttctacagcg                                                   20


<210>  23
<211>  20
<212>  DNA
<213>  Artificial Sequence

<220>
<223>  Primer used for PCR verification of block assembly, Primer ID A11

<400>  23
acgcacgctc ccctgaccat                                                   20


<210>  24
<211>  20
<212>  DNA
<213>  Artificial Sequence

<220>
<223>  Primer used for PCR verification of block assembly, Primer ID A12

<400>  24
ggctctgcgc tgttgaggtc                                                   20


<210>  25
<211>  20
<212>  DNA
<213>  Artificial Sequence

<220>
<223>  Primer used for PCR verification of block assembly, Primer ID B1

<400>  25
gccatagctg ccccaagagc                                                   20

<210>  26
<211>  20
<212>  DNA
<213>  Artificial Sequence

<220>
<223>  Primer used for PCR verification of block assembly, Primer ID B2

<400>  26
gtcgtgcttt ggggcgtacg                                                   20


<210>  27
<211>  20
<212>  DNA
<213>  Artificial Sequence

<220>
<223>  Primer used for PCR verification of block assembly, Primer ID B3

<400>  27
ctccggaacg gtcgcttgga                                                   20


<210>  28
<211>  20
<212>  DNA
<213>  Artificial Sequence

<220>
<223>  Primer used for PCR verification of block assembly, Primer ID B4

<400>  28
tggttgtcac cgacggcggt                                                   20


<210>  29
<211>  20
<212>  DNA
<213>  Artificial Sequence

<220>
<223>  Primer used for PCR verification of block assembly, Primer ID B5

<400>  29
cggcgccgat attggccttc                                                   20


<210>  30
<211>  20
<212>  DNA
<213>  Artificial Sequence

<220>
<223>  Primer used for PCR verification of block assembly, Primer ID B6

<400>  30
cggcgcggtt gtcgaacagt                                                   20


<210>  31
<211>  20

<212>  DNA
<213>  Artificial Sequence

<220>
<223>  Primer used for PCR verification of block assembly, Primer ID B7

<400>  31
ctctcgcgga tcggtccctt                                                   20


<210>  32
<211>  20
<212>  DNA
<213>  Artificial Sequence

<220>
<223>  Primer used for PCR verification of block assembly, Primer ID B8

<400>  32
tcgactccgg ggcgttttcc                                                   20


<210>  33
<211>  20
<212>  DNA
<213>  Artificial Sequence

<220>
<223>  Primer used for PCR verification of block assembly, Primer ID B9

<400>  33
acccttcttg cgacgtgggc                                                   20


<210>  34
<211>  20
<212>  DNA
<213>  Artificial Sequence

<220>
<223>  Primer used for PCR verification of block assembly, Primer ID B10

<400>  34
tcgaagtgaa cctgccgccg                                                   20


<210>  35
<211>  20
<212>  DNA
<213>  Artificial Sequence

<220>
<223>  Primer used for PCR verification of block assembly, Primer ID B11

<400>  35
gcttgttgag cgcggcgaac                                                   20


<210>  36
<211>  20
<212>  DNA
<213>  Artificial Sequence

<220>
<223>  Primer used for PCR verification of block assembly, Primer ID B12

<400>  36
ttttgcccag gacgccgcag                                                   20


<210>  37
<211>  20
<212>  DNA
<213>  Artificial Sequence

<220>
<223>  Primer used for PCR verification of block assembly, Primer ID C1

<400>  37
cagatagccg cgagcgtacg                                                   20


<210>  38
<211>  20
<212>  DNA
<213>  Artificial Sequence

<220>
<223>  Primer used for PCR verification of block assembly, Primer ID C2

<400>  38
gcgatgtgac cagcgtccag                                                   20


<210>  39
<211>  20
<212>  DNA
<213>  Artificial Sequence

<220>
<223>  Primer used for PCR verification of block assembly, Primer ID C3

<400>  39
tcgatgtcga cggcggtcag                                                   20


<210>  40
<211>  20
<212>  DNA
<213>  Artificial Sequence

<220>
<223>  Primer used for PCR verification of block assembly, Primer ID C4

<400>  40
atccacaacg ccgcctgcga                                                   20


<210>  41
<211>  20
<212>  DNA
<213>  Artificial Sequence

<220>
<223>  Primer used for PCR verification of block assembly, Primer ID C5

<400>  41
tcagcatgat ccgggcgtgc                                                   20


<210>  42
<211>  20
<212>  DNA
<213>  Artificial Sequence

<220>
<223>  Primer used for PCR verification of block assembly, Primer ID C6

<400>  42
gtcggtcgca ggatgacgct                                                   20


<210>  43
<211>  20
<212>  DNA
<213>  Artificial Sequence

<220>
<223>  Primer used for PCR verification of block assembly, Primer ID D1

<400>  43
gacgcggtta tcgatggcga                                                   20


<210>  44
<211>  20
<212>  DNA
<213>  Artificial Sequence

<220>
<223>  Primer used for PCR verification of block assembly, Primer ID D2

<400>  44
ggtttcgggc ggttgtccat                                                   20


<210>  45
<211>  20
<212>  DNA
<213>  Artificial Sequence

<220>
<223>  Primer used for PCR verification of block assembly, Primer ID D3

<400>  45
agcagcatgg cggggaagtt                                                   20


<210>  46
<211>  20
<212>  DNA
<213>  Artificial Sequence

<220>
<223>  Primer used for PCR verification of block assembly, Primer ID D4

<400>  46
ccacctacag ctgcttgcca                                                   20

<210>  47
<211>  20
<212>  DNA
<213>  Artificial Sequence

<220>
<223>  Primer used for PCR verification of block assembly, Primer ID D5

<400>  47
cccaccacga caatgatgcg                                                   20


<210>  48
<211>  20
<212>  DNA
<213>  Artificial Sequence

<220>
<223>  Primer used for PCR verification of block assembly, Primer ID D6

<400>  48
ccacaagatc tggcgcggta                                                   20


<210>  49
<211>  20
<212>  DNA
<213>  Artificial Sequence

<220>
<223>  Primer used for PCR verification of block assembly, Primer ID D7

<400>  49
actgagctac ccaggcatcc                                                   20


<210>  50
<211>  20
<212>  DNA
<213>  Artificial Sequence

<220>
<223>  Primer used for PCR verification of block assembly, Primer ID D8

<400>  50
tcgagacgaa ggtcggcttc                                                   20

## Description

### Background

During the past decade, high-throughput DNA sequencing has transformed every aspect of biological sciences and medicine. Today, we are at the dawn of a new era where biological sciences transform from a knowledge-oriented discipline towards application-related engineering of complex biological systems thereby multiplying and capitalizing its highly innovative technological potential to produce diverse molecules with application in medicine, agriculture, material sciences and sustainable food and bioenergy production.

The recent advances in low-cost de novo DNA synthesis technologies provides now for the first time the capabilities to program biological functions by writing long DNA molecules. In future, de novo synthesis of DNA will have even larger transformative impacts on biology and medicine than the genomic revolution of sequencing. During this transformation, new enabling technologies, such as the herein proposed evolution-guided multiplexed genome assembly process, will be key for cost and time efficient manufacturing of synthetic DNA designs to accelerate bio-engineering of complex biological systems.

Despite recent technological break through in de novo DNA synthesis capabilities chromosome assembly and editing tools, fast-paced de novo DNA synthesis still represents the major rate-limiting step of synthetic biology towards efficient manufacturing of platform organisms with fully defined genetic makeup.

Silicon and chips-based approaches for de novo DNA synthesis now enable en-masse manufacturing of short double stranded DNA sequences (as exemplified by technologies used by Twist, Gen9, Thermo-Fisher). These approaches enables simultaneous production of tens of thousands of short oligonucleotides that are assembled into 1 kb long double stranded DNA molecules and, in a next iteration, subsequently joined into higher-order assemblies. However, due to the miniaturisation and limits of the solid-phase chemistry, advanced low-cost oligo manufacturing technologies do not guarantee that every DNA block can be manufactured in a streamlined manner.

The cornerstone of current large scale DNA manufacturing process still follows design principles adopted from classical chemical synthesis: First define the sequence of the desired DNA molecule and then build an exact copy upon series of sequential chemical reactions. During de novo DNA synthesis, the structure (base pair sequence) of the DNA molecule (the design) is kept constant. Side products or intermediates that are not identical to the initial sequence design (or parts thereof) are discarded during subsequent separation and sequencing process. Synthesis errors during oligo synthesis and polymerase chain assembly (PCA) reaction require repetition, optimisation and refinement of reaction conditions until sufficient yields are achieved to proceed with subsequent higher order DNA assembly steps. Due to the intrinsic hierarchical nature, this process strictly depends on successful manufacturing of each individual building block from the preceding assembly level. Therefore, engineering of synthetic pathways, gene clusters and entire genomes composed of hundreds to thousands of DNA blocks quickly becomes a insurmountable problem, as even one single missing DNA block impedes hierarchical assembly and, thus, prevents completion of the DNA design. As a consequence, current genome manufacturing is delayed till every difficult to synthesise DNA block has been obtained during iterative cycles of de novo DNA synthesis attempts.

### Description of the invention

Based on the above described background, it is the objective of the present invention to provide a process for generating large DNA constructs that may comprise whole pathways, gene clusters or entire genomes.

This objective is attained by a process having the features of claim 1. Preferred embodiments are state in the dependent claims and the description below.

According thereto, a first aspect of the invention relates to a process for manufacturing a large DNA construct of interest. The process comprises the steps of:
- providing an *in silico* template DNA construct comprising a plurality of genetic elements;
- subjecting the *in silico* template DNA construct to a computational optimization step, wherein one or more sequences inhibiting de novo DNA synthesis are removed from the template *in silico* DNA construct by neutral sequence change, particularly by neutral codon replacement in case of being comprised within one or more protein coding sequences or by neutral base substitution insertion, deletion or synonymous sequence replacement in case of being comprised within one or more intergenic sequences, yielding an optimized *in silico* DNA construct, provided that start codons are not removed or replaced;
- partitioning the optimized *in silico* DNA construct into a plurality of original *in silico* assembly units in a partitioning step, wherein the optimized *in silico* DNA construct is partitioned such that in each case two adjacent members of the plurality of original *in silico* assembly units share a terminal homology region, wherein one terminal homology region differs from any other;
- subjecting each member of the plurality of original *in silico* assembly units to a computational synonymous sequence recoding step, wherein
   - one or more synonymous *in silico* assembly units are generated for each member of the plurality of original *in silico* assembly units by neutral sequence change, provided that no terminal homology region or start codon is altered, and
   - an *in silico* assembly variant pool comprising the member of the plurality of *in silico* original assembly units and the one or more synonymous *in silico* assembly units is generated, thereby yielding a library of *in silico* variant pools;
- de novo synthesizing one or more members of each *in silico* assembly variant pool of said library of *in silico* variant pools, thereby yielding a library of nucleic acid assembly units; and
- assembling the library of nucleic acid assembly units into the DNA construct of interest *in vitro* or *in vivo* in an assembly step.

Whenever a construct or an assembly unit is termed as "*in silico*" it should be understood in the context of the present specification that the respective construct or assembly unit exists in form of a digital sequence, e.g. encoded in a computer readable format.

Particularly, whenever two adjacent assembly units share a terminal homology region it should be understood that both adjacent assembly units comprise the respective terminal homology region upon which the two assembly units are assembled.

The term "neutral sequence change" in the context of the present specification particularly refers to a change in the sequence that does not affect the biological function of the respective sequence, e.g. causing only silent mutations.

Non-limiting examples for neutral sequence changes include
- neutral codon replacement within protein coding sequences, and
- neutral base substitution, insertion, or deletion or synonymous sequence replacement within intergenic sequences.

The term "intergenic sequence" in the context of the present specification particularly refers to a non-coding stretch of DNA located between two genes.

The term "neutral codon replacement" in the context of the present specification refers to the exchange of a codon by a different codon encoding the same amino acid residue within a protein coding sequence of the DNA construct of interest, or within an in silico assembly unit.

The term "synonymous sequence replacement" in the context of the present specification particularly refers to the replacement of one or more intergenic sequences within the template *in silico* template by one or more sequences that provides a similar biological function.

The term "neutral base substitution, insertion or deletion" in the context of the present specification particularly refers to a base substitution, insertion or deletion that does not affect the biological function of the respective sequence.

Particularly, the one or more sequences inhibiting de novo DNA synthesis are removed by replacing them with one or more synonymous sequences not inhibiting de novo synthesis, particularly encoding the same polypeptide or providing a similar biological function, wherein the one or more synonymous sequences are generated by neutral sequence change, e.g. neutral codon replacement within protein coding sequences or neutral base substitution, insertion or deletion or synonymous sequence replacement within intergenic sequences.

The skilled person understands that each of the above mentioned original *in silico* assembly units and accordingly each of the one or more synonymous *in silico* assembly units except of the initial and terminal assembly unit comprise two homology regions, upon which the respective assembly unit can be assembled with the preceding assembly unit and the subsequent assembly unit.

Non-limiting examples of sequences that inhibits de novo DNA synthesis include sequence with a high GC content, particularly higher than of 50 %, homopolymeric sequences having a length of 6 bp or above, di- and trinucleotide repeats, direct repeats and longer hairpins, particularly having a length in range of 8 bp to 12 bp or above.

A non-limiting example for *in vitro* assembly is the Gibson assembly, wherein the nucleic acid assembly units assembled upon the terminal homology region. A non-limiting example for *in vivo* assembly is the yeast assembly, wherein a yeast cell is transformed with the nucleic acid assembly units, particularly by means of a suitable vehicle such as a vector, and the nucleic acid assembly units are assembled within the yeast cell.

Advantageously, the process of the invention overcomes the limitation of known methods regarding assembly units that are hardly or even not at all synthesisable by the provision of one more synonymous assembly units, by which the probability of a successful de novo synthesis of all required assembly units for a successful assembly is greatly increased.

Furthermore, the process of invention not only allows the generation of large DNA constructs, also the generation of variants thereof is possible by non-neutral codon or non-synonymous sequence replacement in the computational optimization step and/or the computational synonymous sequence recoding step.

Accordingly, a second aspect of the invention relates to a process for manufacture a variant of a DNA construct of interest, comprising the steps of:
- providing an original *in silico* DNA construct comprising a plurality of genetic elements;
- subjecting the original *in silico* DNA construct to a computational optimization step, wherein one or more sequences inhibiting de novo DNA synthesis are removed from the template *in silico* DNA construct by neutral sequence change, yielding an optimized *in silico* DNA construct, provided that start codons are not removed or replaced;
- partitioning the optimized *in silico* DNA construct into a plurality of original *in silico* assembly units in a partitioning step, wherein the optimized *in silico* DNA construct is partitioned such that in each case two adjacent members of the plurality of original *in silico* assembly units share a terminal homology region, wherein one terminal homology region differs from any other;
- subjecting each member of the plurality of original *in silico* assembly units to a computational mutating sequence recoding step or a computational synonymous sequence recoding step, wherein
   - in the computational mutating sequence recoding step, one or more mutant *in silico* assembly units are generated for one or more members of the plurality of original *in silico* assembly units by non-neutral sequence change, provided that no terminal homology region or start codon is altered, and an *in silico* assembly mutant pool comprising the one or more mutant *in silico* assembly units is generated, thereby yielding a respective library of *in silico* mutant pools; and
   - in the computational synonymous recoding step, one or more synonymous *in silico* assembly units are generated for each member of the plurality of original *in silico* assembly units not being subjected to the computational mutating sequence recoding step by neutral sequence change, provided that no terminal homology region or start codon is altered, and an *in silico* assembly variant pool comprising the member of the plurality of original *in silico* assembly units and the one or more synonymous *in silico* assembly units is generated, thereby yielding a respective library of *in silico* variant pools;
- de novo synthesizing one or more members of each *in silico* assembly variant pool of the library of in silico variant pools and one or more members of each in silico mutant pool of the library of *in silico* mutant pools, thereby yielding a library of nucleic acid assembly units; and
- assembling the library of nucleic acid assembly units into the variant of a DNA construct of interest *in vitro* or *in vivo* in an assembly step.

The term "non-neutral sequence change" in the context of the present specification particularly refers to a change in the sequence that does affect the biological function of the respective sequence.

Non-limiting examples for non-neutral sequence changes include
- non-neutral codon replacement within protein coding sequences,
- introducing of frame shifts by base insertions or deletions within open reading frames in protein coding sequences, and
- non-neutral base substitutions, insertions non-synonymous sequence replacement within intergenic sequences.

The term "non-neutral codon replacement" in the context of the present specification refers to the exchange of a codon by a different codon encoding a different amino acid residue within a protein coding sequence of the DNA construct of interest or within an *in silico* assembly unit.

The term "non-synonymous sequence replacement" in the context of the present specification particularly refers to the replacement of one or more intergenic sequences within the template *in silico* template or within an *in silico* assembly unit that does not provide a similar biological function.

The term "non-neutral base substitution, insertion or deletion" in the context of the present specification particularly refers to a base substitution, insertion or deletion that affects the biological function of the respective sequence.

In certain embodiments, one or more sequences comprised within one or more protein coding sequences and inhibiting de novo DNA synthesis are removed from the template *in silico* DNA construct by neutral codon replacement in the computational optimization step.

In certain embodiments, one or more sequences comprised within one or more intergenic sequences and inhibiting de novo DNA synthesis are removed from the template *in silico* DNA construct by neutral base substitution, insertion, or deletion or synonymous sequence replacement in the computational optimization step.

An alternative process for manufacturing a variant of a DNA construct of interest comprises the steps of:
- providing an original *in silico* DNA construct comprising a plurality of genetic elements;
- subjecting the original *in silico* DNA construct to a computational mutagenesis step, wherein one or more sequences inhibiting de novo DNA synthesis are removed from the original *in silico* DNA construct by non-neutral sequence change, yielding an optimized mutant *in silico* DNA construct, provided that start codons are not removed or replaced;
- partitioning the optimized mutant *in silico* DNA construct into a plurality of original *in silico* assembly units in a partitioning step, wherein the optimized mutant *in silico* DNA construct is partitioned such that in each case two adjacent members of the plurality of original *in silico* assembly units share a terminal homology region, wherein one terminal homology region differs from any other;
- subjecting each member of the plurality of original *in silico* assembly units to a computational synonymous sequence recoding step, wherein
   - one or more synonymous *in silico* assembly units are generated for each member of the plurality of original *in silico* assembly units by neutral sequence change, provided that no terminal homology region or start codon is altered, and
   - an *in silico* assembly variant pool comprising the member of the plurality of original *in silico* assembly units and the one or more synonymous *in silico* assembly units is generated, thereby yielding a respective library of *in silico* variant pools;
- de novo synthesizing one or more members of each *in silico* assembly variant pool of the library of in silico variant pools, thereby yielding a library of nucleic acid assembly units; and
- assembling the library of nucleic acid assembly units into the variant of a DNA construct of interest *in vitro* or *in vivo* in an assembly step.

In certain embodiments, one or more sequences comprised within one or more protein coding sequences and inhibiting de novo DNA synthesis are removed from the original *in silico* DNA construct by non-neutral codon replacements or base deletion within one or more protein coding sequences in the computational mutagenesis step.

In certain embodiments, one or more sequences comprised within one or more intergenic sequences and inhibiting de novo DNA synthesis are removed from the original *in silico* DNA construct by non-neutral base substitution, insertion or deletion or by non-synonymous replacement in the computational mutagenesis step.

Alternatively, such variant may be generated *in silico* by non-neutral sequence changes such as non-neutral codon replacement or non-synonymous sequence replacement with a original DNA construct, yielding an *in silico* mutant DNA construct, which is then subjected to a process according to the above aspect of the invention, yielding the mutant DNA construct in form of a corresponding nucleic acid.

Accordingly, a further alternative process for manufacture a variant of a DNA construct of interest comprises the steps of:
- providing a template *in silico* DNA construct comprising a plurality of genetic elements;
- subjecting the template *in silico* DNA construct a computational mutagenesis step, wherein one or more sequences within the *in silico* template DNA construct are altered by non-neutral sequence change, yielding a mutant *in silico* DNA construct
- subjecting the mutant *in silico* DNA construct to a computational optimization step, wherein one or more sequences inhibiting de novo DNA synthesis are removed from the template *in silico* DNA construct by neutral sequence change, yielding an optimized mutant *in silico* DNA construct, provided that start codons are not removed or replaced;
- partitioning the optimized mutant *in silico* DNA construct into a plurality of original *in silico* assembly units in a partitioning step, wherein the optimized mutant *in silico* DNA construct is partitioned such that in each case two adjacent members of the plurality of original *in silico* assembly units share a terminal homology region, wherein one terminal homology region differs from any other;
- subjecting each member of the plurality of original *in silico* assembly units to a computational synonymous sequence recoding step, wherein
   - one or more synonymous *in silico* assembly units are generated for each member of the plurality of original *in silico* assembly units by neutral sequence change, provided that none terminal homology region or start codon is altered, and
   - an *in silico* assembly variant pool comprising said member of said plurality of original assembly units and the one or more synonymous *in silico* assembly units is generated, thereby yielding a library of *in silico* variant pools
- de novo synthesizing one or more members of each *in silico* assembly variant pool of the library of *in silico* variant pools, thereby yielding a library of nucleic acid assembly units; and
- assembling the library of nucleic acid assembly units into the variant of a DNA construct of interest *in vitro* or *in vivo* in an assembly step.

In certain embodiments, one or more sequences comprised within one or more protein coding sequences are altered by non-neutral codon replacements or base deletion within one or more protein coding sequences in the computational mutagenesis step.

In certain embodiments, one or more sequences comprised within one or more intergenic sequences are altered by non-neutral base substitution, insertion or deletion or by non-synonymous sequence replacement in the computational mutagenesis step.

In certain embodiments, sequences with a CG content equal or above 50 %, 60 %, 70 %, 80 % or 85 % and having a length in range of 21 base pairs to 99 base pairs are removed from the template *in silico* DNA construct. In certain embodiments, sequences with a CG content equal or above 70 % and having a length of 21 base pairs are removed from the template *in silico* DNA construct. In certain embodiments, sequences with a CG content equal or above 85 % and having a length of 99 base pairs are removed from the template *in silico* DNA construct.

In certain embodiments, the library of nucleic acid assembly units is amplified in an amplification step before the assembly step, yielding an amplified library of nucleic acid assembly units, wherein the amplified library of nucleic acid assembly units is assembled into the DNA construct of interest or the variant thereof in the assembly step.

In certain embodiments, the one or more members of each *in silico* assembly unit variant or mutant pool are synthesized as double-stranded DNAs, wherein particularly the double-stranded DNAs are attached to a solid support or are present in solution.

In certain embodiments, a first detachable adapter sequence is added to one end of each member of each *in silico* assembly variant or mutant pool, and a second detachable adapter sequence is added to the other end of each member of each *in silico* assembly variant or mutant pool, wherein
- the first detachable adapter sequence and the second detachable adapter sequence have different sequences, and wherein optionally a first primer capable of annealing to the first detachable adapter sequence and a second primer capable of annealing to the second detachable adapter sequence are used in the amplification step, and
- the first detachable adapter sequence and the second detachable adapter sequence are removed from each member of the library of nucleic acid assembly units or the amplified library of nucleic acid assembly units before the assembly step.

The skilled person understands that the first detachable adapter sequences and the second detachable adapter sequences added to an *in silico* assembly units are synthesized as nucleic acid sequences attached to the corresponding nucleic acid assembly unit.

In certain embodiments, the first detachable adapter sequence comprises a first primer binding region and a first cleavage site, wherein the first cleavage site is arranged between the first primer binding region and the one end of each member of each *in silico* assembly variant or mutant pool.

In certain embodiments, the second detachable adapter sequence comprises a second primer binding region and a second cleavage site, wherein the second cleavage site is arranged between the second primer binding region and the other end of each member of each *in silico* assembly variant or mutant pool.

In certain embodiments, the first cleavage site and the second cleavage site are specifically recognizable by different endonucleases.

In certain embodiments, the first primer consist of or comprise a nucleic acid sequence being at least 80 %, 85 %, 90 %, 95 %, 99 % or 100 % identical or complementary to the fist primer binding region. In certain embodiments, the second primer consist of or comprise a nucleic acid sequence being at least 80 %, 85 %, 90 %, 95 %, 99 % or 100 % identical or complementary to the second primer binding region.

In certain embodiments, the DNA construct of interest or the variant thereof is a linear nucleic acid molecule, a circular nucleic acid molecule such as a plasmid, or an artificial chromosome.

In certain embodiments, DNA construct of interest has a length of at least 10,000 base pairs. In certain embodiments, DNA construct of interest has a length of at least 1000,000 base pairs.

In certain embodiments, each member of the plurality of original *in silico* assembly units independently of each other has a length in range of 500 base pairs to 3,000 base pairs.

In certain embodiments, each of the terminal homology regions independently from each other has a length a 15 base pairs to 35 base pairs.

In certain embodiments, the genetic element is select from an operon, a promoter, an open reading frame, an enhancer, a silencer, an exon, an intron, or a gene.

In certain embodiments, the DNA construct of interest, the original *in silico* DNA construct or the template *in silico* DNA construct comprises or consists of one or more gene clusters, or a whole genome. In certain embodiments, the DNA construct of interest, the original *in silico* DNA construct or the template *in silico* DNA construct comprises a plurality of genetic elements corresponding to one or more metabolic pathways.

In certain embodiments, the template DNA construct or original DNA construct is naturally occurring or artificial.

Such artificial DNA construct may originate from a naturally occurring nucleic acid such as a gene cluster or a genome, in which one or more foreign genetic elements such as genes, promoters, operons, or open reading frames have be incorporated, and/or naturally occurring genetic elements have been replaced and/or deleted. Such artificial DNA construct may also be a mosaic of a plurality of genetic elements originating from a plurality of different organisms.

In certain embodiments, the template *in silico* DNA construct is a variant of a functional DNA construct of natural or artificial origin, particularly meaning a DNA construct comprised of functional genetic elements, wherein one or more genetic elements are rendered non-functional by insertion or deletion of bases or sequences, or inversion of sequences or non-neutral codon replacements.

In certain embodiments, the terminal homology region is comprised within a protein coding sequence, wherein said terminal homology region starts in frame with the protein coding sequence. In certain embodiments, the terminal homology region is comprised within an intergenic sequence.

In certain embodiments, the partitioning step comprises
- partitioning the optimized *in silico* DNA construct or the optimized mutant *in silico* DNA construct into a plurality of *in silico* segment assembly units, wherein in each case two adjacent *in silico* segments assembly units share a segment terminal homology region, wherein particularly one segment terminal homology region differs from any other;
- partitioning each member of the plurality of *in silico* segment assembly units into a plurality of *in silico* block assembly units, wherein in each case two adjacent block assembly units share a block terminal homology region, wherein particularly one block terminal homology region differs from any other; and
- partitioning each member of the plurality of *in silico* block assembly units into a plurality of *in silico* subblock assembly units, wherein in each case two adjacent subblock assembly units share a subblock terminal homology region, thereby yielding the plurality of original *in silico* assembly units as described above.

In certain embodiments, the assembly step comprises
- pooling and assembling members of the library of nucleic acid assembly units or the amplified library of nucleic acid assembly units corresponding to an *in silico* block assembly unit into a nucleic acid block assembly unit, respectively, yielding a plurality of nucleic acid block assembly units;
- pooling and assembling nucleic acid block assembly units corresponding an *in silico* segment assembly unit into a nucleic acid segment assembly unit, respectively, yielding a plurality of nucleic acid segment assembly units; and
- pooling and assembling the nucleic acid segment assembly units into the DNA construct of interest or a variant thereof.

In certain embodiments, the first detachable adapter sequence is or comprises a segment adapter sequence, and the second detachable adapter sequence is or comprises a block adapter sequence, wherein
- members of each *in silico* assembly variant or mutant pool corresponding to the same *in silico* segment assembly unit have the same segment adapter sequence,
- members of each *in silico* assembly variant or mutant pool corresponding to the same *in silico* block assembly unit have the same block adapter sequence,
- each segment adapter sequence differs from each other, and
- each block adapter sequence differs from each other

In certain embodiments, the segment adapter sequence is added to the 5' end of the respective member of the respective *in silico* assembly variant or mutant pool, and the block adapter sequence is added to the 3' end of the respective member.

In certain embodiments, each member of the plurality of *in silico* segment assembly units independently of each other has a length in the range of 10,000 base pairs to 50,000 base pairs.

In certain embodiments, each member of the plurality of *in silico* block assembly units independently of each other has a length in range of 2,000 base pairs to 10,000 base pairs.

In certain embodiments, each of the segment terminal homology regions has independently from each other a length in the range of 35 base pairs to 200 base pairs.

In certain embodiments, each of the block terminal homology regions has independently from each other a length in the range of 35 base pairs to 90 base pairs.

The invention is further illustrated by the following detailed description of certain embodiments, examples and figures, from which further embodiments and advantages can be drawn. The examples are meant to illustrate the invention but not to limit its scope.

### Short description of the figures

- Fig. 1: shows the workflow for the evolution-guided multiplexed genome assembly process.
- Fig. 2: shows a map of the 773'851 base pair long tamed genome design, and the partitioning design indicating synthesis success rates by current methods.

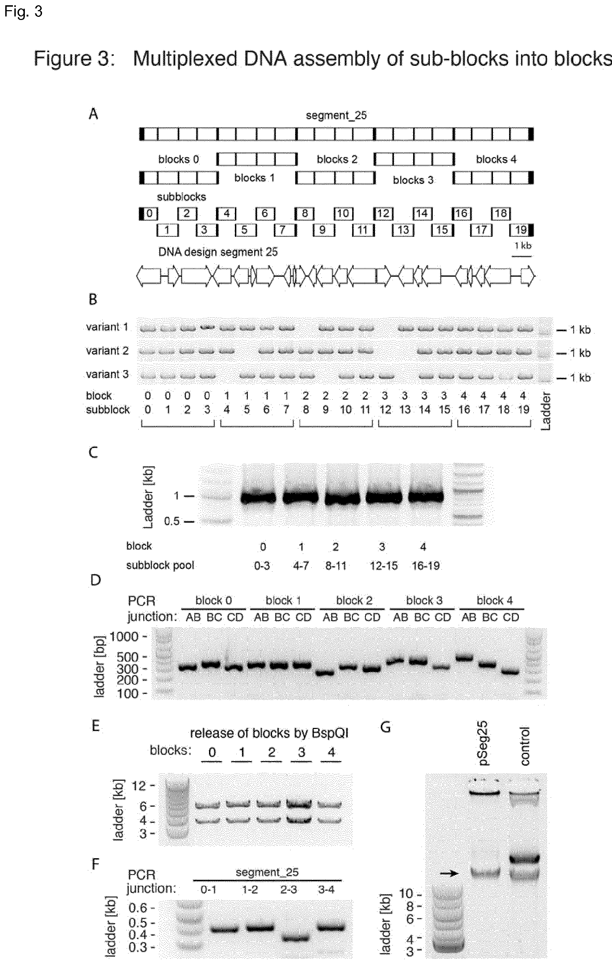

- Fig. 3: shows multiplexed DNA assembly of sub-blocks into blocks (A) Overview of the partitioning design. (B) Overview of de novo DNA synthesis yield of subblock design variants. Barcoded subblocks were PCR amplified and separated on a 1% agarose gel. De novo DNA synthesis failed for design1: sb 8, sb12; design 2: sb5, sb12, sb13; design 3: sb 4, sb9, sb13. (C) Pools of subblocks for block assembly generated by barcode specific PCR amplification. Each PCR reaction product contains the set of all subblocks design variants for a particular block assembly that have successfully been synthesized by PCA. (D) Multiplexed block assembly reactions of segment 25. Correct assemblies for all 5 blocks are confirmed by PCR across subblock junctions. Each block was assembled from 4 subblocks named A-D, and junctions tested by PCR are labelled accordingly AB, BC, CD. (E) Release of blocks from the cloning vector using restriction digestion. The lower band corresponds to the block fragments of 4kb in size. (F) PCR verification of assembly of blocks into a 20kb segment amplifying block junctions. (G) Verification of the size of the assembled segment 25 construct in the destination vector pMR10Y producing pSeg25. The size of the super-coiled plasmid pSeg25 is compared to a super-coiled reference plasmid pMR10Y carrying a 19kb insert (white arrow).
- Table 1: DNA synthesis yield of the tamed genome partitioned in 236 blocks of ∼4kb.
- Table 2: Base substitution rates between subblock designs variants.
- Table 3: De novo DNA synthesis yield of subblock design variants from segment 25.
- Table 4: Efficiency of block assembly reactions using pools of subblock variants.
- Table 5: Adaptor sequences used for partitioning.
- Table 6: Barcode primers used subpool PCR amplification.
- Table 7: Primers used for PCR verification of block assembly.
- Table 8: List of strains.

### Detailed description of certain embodiments

The invention achieves leveraging de novo DNA synthesis and engineering to the genomic scale, thereby reducing time and costs for bio-systems design through a scalable DNA synthesis process termed evolution-guided multiplexed DNA assembly. The process solves the problem of manufacturing large-scale DNA constructs in a hierarchical manner from numerous small double-stranded DNA blocks that each cannot be produced with 100% success rate.

Instead of building a single DNA sequence design, evolution-guided multiplexed DNA assembly, employs multiple synonymous DNA sequence variants in parallel and selects in a combinatorial assembly approach for those sequence variants with the best synthesis and assembly feasibility.

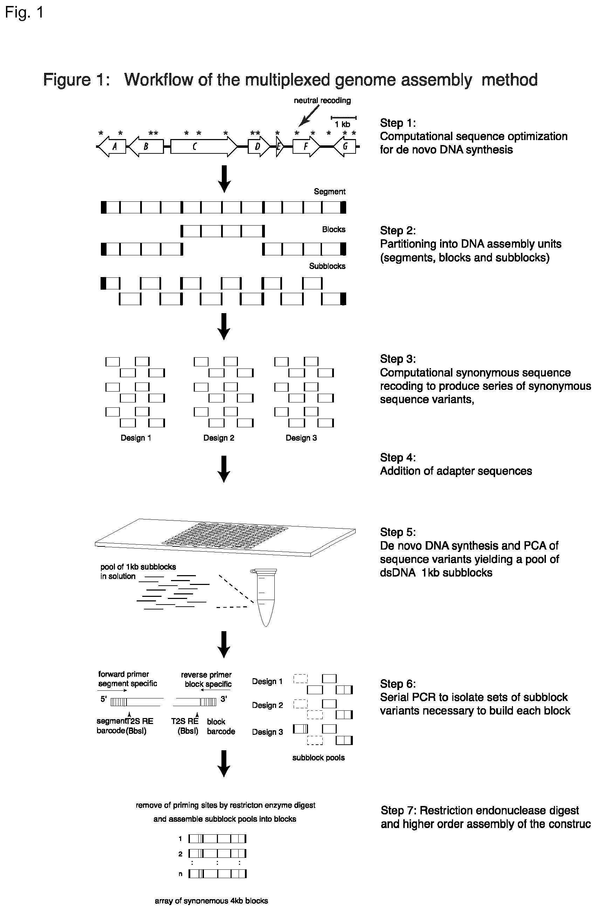

In certain embodiments, the multiplexed genome assembly process of the invention is based on a 7 steps process (Figure 1). The major stages of the process are i) computational optimization of the DNA design (referred to as DNA construct of interest above) for de novo DNA synthesis, ii) partitioning into DNA assembly units (segments, blocks and subblocks, referred to as original *in silico* assembly units above), iii) computational synonymous sequence recoding to produce series of synonymous sequence variants (referred to as synonymous *in silico* assembly units above), iv) addition of adapter sequences to subblock design variants, v) de novo DNA synthesis of synonymous sequence variants pools, vi) serial PCR to isolate sets of subblock variants necessary to build each block, vii) removal of terminal PCR barcode sequences and higher order assembly of the construct.

The key principle of the invention is that DNA designs are sequence optimized and partitioned into synonymous variants that serve as redundant assembly units for higher order DNA assembly. Thus the DNA synthesis does not critically depend on successful synthesis of all building units

*First Step: Computational optimization of the design for de novo DNA synthesis -* The DNA sequence design (in size up to entire artificial genomes) is optimized for de novo DNA synthesis to yield a synthesis-optimized DNA design.

In certain embodiments, the DNA sequence design represents a nucleic acid molecule, a plasmid or artificial chromosome(s).

In certain embodiments, the DNA sequence design comprises more than (>) 10.000 bp, particularly > 1.000.000 bp.

Using the Genome Calligrapher Software algorithm or similar computational algorithms, protein-coding sequences of the said DNA sequence design are refactored by neutral recoding (synonymous codon replacement) to erase disallowed sequence patterns known to inhibit de novo DNA synthesis. Sequence design and methods of sequence refactoring are described in EP15195390.8, hereby incorporated by reference in its entirety. The Genome Calligrapher Software algorithm for DNA refactoring by neutral recoding, codon optimization and methods of their use are described in (CHRISTEN, M., DEUTSCH, S., & CHRISTEN, B. (2015). Genome Calligrapher: A Web Tool for Refactoring Bacterial Genome Sequences for de Novo DNA Synthesis. ACS Synthetic Biology, 4(8), 927-934. http://doi.org/10.1021/acssynbio.5b00087), hereby incorporated by reference in its entirety.

*Second Step: Partitioning into DNA assembly units -* The synthesis-optimized DNA design is partitioned into DNA units (segments, blocks, subblocks) used for hierarchical assembly. Up to three assembly levels are integrated. At the first level, sets of subblocks are assembled into blocks. At the second assembly level sets of blocks are further assembled into segments, which are ultimately assembled into the final large-scale DNA construct. With increase in assembly level, DNA assembly units increase in size and ideally are for subblocks in the range of 500-3'000 bp, for blocks in the range of 2'000-10'000 bp, and for segments in the range of 10'000-50'000 bp. Across the entire partitioning design, short terminal homology regions (THRs) (from 15 to 200 bp in size) are defined between adjacent assembly units. These regions provide terminal sequence homologies used for higher-order assembly known in the art and use to concatenate adjacent assembly units into higher order constructs. Boundaries for THRs are defined according to following design rules:
- Boundaries for said THRs reside either inside intergenic sequences or within protein coding DNA sequences (CDS).
- Boundaries of each assembly unit are set inframe if THRs fall within CDS.

An aspect of the invention relates to a computational process for partitioning large multi-kilobase DNA sequences, wherein a software algorithm (Genome Partitioner) is used to perform DNA sequence partitioning into hierarchical assembly levels and define terminal homology regions according to the above specified design rules. Three assembly levels are integrated into said algorithm:
The DNA sequence partitioning algorithm uses an annotated DNA sequence file (GenBank file) as input and comprises the steps of:
   a) Partitioning of the DNA sequence into DNA segments with user defined segment size (ideally in the range of 10 to 100 kb with a size deviation smaller than 10%, including segment THRs). Each segment shares terminal homology regions to the previous (5') segment (ideally in the range of 35-200 bp). Boundaries of THRs that fall within coding sequences are adjusted to fit into corresponding reading frames. This adjustment is done during creation and optimisation of THRs. DNA segments carry adjacent 5' and 3' terminal adaptor sequences covering homologies to a destination vector and optionally contain linker sequences for restriction endonuclease digest, cloning or higher-order assembly in yeast. Sequence records of segments (including terminal adaptors) are written in a fasta file and segment boundaries without adaptor are annotated in a GenBank output file. In certain embodiments, THR at the segment level are optimized according to THR design rules similar to the THR design rules at the block level as specified in (b) below.
   b) The DNA segments are further subdivided into DNA blocks using the following design rules: DNA blocks are of a user defined size (ideally in the range of 2 to 10 kb, including length of segment and block THRs and adaptor sequences, and are of uniform size with a size deviation smaller than 10%). DNA blocks overlap with adjacent blocks by a user-defined block THR (ideally in the range of 35 to 90 bp). Boundaries of block THRs are adjusted to fit in corresponding reading frames. DNA blocks carry adjacent adaptor sequences covering homologies to i) a destination vector and ii) optionally contain linker sequences for restriction endonuclease digest and cloning to a destination vector
   c) Terminal homology regions of assembly units at the block level are analysed for presence of sequence features that interfere with homologous end-joining known in the art and use to concatenate adjacent assembly units. Hairpins and direct repeat sequences of repeat size larger than a user-specified limit (8bp) within THRs are removed by shifting the THR upstream or downstream to no longer include the repeat sequence or any additional repeat sequence (non-unique sequence pattern) and readjust block-boundaries accordingly. Identical substrings occurring multiple times (i.e. non-unique sequences) within THR regions of DNA blocks of each segment are calculated. The largest identical substring occurring within multiple THRs at the block level is identified and removed by generating a set of partitioning variants with shifted THRs that no longer include the problematic non-unique sequence pattern. These partitioning design variants are iteratively evaluated for occurrence of repeat, hairpins and multiple occurrences of substrings within multiple THR. A metric is then used to identify the optimal partitioning design variant that i) shows absence of repeats and ii) no occurrence of non-unique sequences and iii) requires the least repositioning of THR regions. Furthermore, for optimizing the THR, the block size is not allowed to deviate more than 10% from the mean block size as provided by the user. The optimal partitioning design is selected and the corresponding block-boundaries are adjusted. The THR optimization is repeated until a user defined lower size limit for identical substrings (8bp) is reached. THRs within protein-coding sequences are adjusted to fit into the corresponding reading frames on both ends. This adjustment is done during creation and optimisation of the THR of each block. After completing the DNA block partitioning, sequence records of blocks are written in a fasta file (adapters included) and block boundaries are annotated in the GenBank outputfile (without block adapters).
   d) The DNA blocks are further subdivided into DNA subblocks using the following design rules: DNA subblocks overlap adjacent subblocks by a user-defined THR (ideally in the range of 15 to 35 bp). No subblock deviates by more than 10% from the user defined maximal subblock size (ideally in the range of 500 to 3000 bp, including length of segment, block and subblock THRs and corresponding adaptor sequences).
   e) Terminal homology regions of DNA subblocks are optimized according to the same routine as employed in c) for THR optimization at the block level. DNA subblocks carry adjacent adaptor sequences covering homologies to a destination vector and optionally contain linker sequences for restriction endonuclease digest for subcloning. Subblocks are written in a fasta file (subcloning adapters and PCR adaptors included) and subblocks are annotated in the GenBank outputfile (without subblock adapters).
      In certain embodiments 5' and 3' adaptor sequences contain specific primer annealing sites that allow parallel PCR amplification of sets of DNA units for higher order assembly.
      In certain embodiments, 5' and 3' adaptor sequences may be omitted if stitching oligos are used for subsequent assembly of DNA units.

*Third Step: Computational synonymous sequence recoding to produce series of synonymous sequence variants -* The partition-optimized DNA design is sequence recoded to produce a set of (n) synonymous sequence variants. Thereby, codons within protein-coding sequences are substituted with synonymous codons. In certain embodiments, variants within intergenic sequences are generated upon introducing base-substitutions, insertions or deletions or replacing the intergenic sequence with a synonymous sequence that covers similar biological functions. Regions where THR have been assigned for the assembly process are excluded from recoding and remain unchanged in sequence. The polypeptide sequence information within each protein coding sequence is encoded by a series of 61 nucleotide triplets for 20 amino acids. This redundancy of the genetic code allows a particular codon to be replaced by synonymous ones that still code for the same amino acid. Through the process of recoding, a set of sequence variants is produced that encode for the same proteins but differ in nucleotide sequence. The Genome Calligrapher Software algorithm for DNA refactoring, codon optimization and methods of their use are described in (CHRISTEN, M., et al. http://doi.org/10.1021/acssynbio.5b00087), hereby incorporated by reference in its entirety.

*Fourth step: Addition of adapter sequences to subblock design variants-* After variant generation sequences of all subblock are retrieved from each design and adapter sequences are added. Adapter sequences are appended to the 3' and 5' termini to facilitate release of partitioning units from propagation vectors and to permit integration of assembled units into destination vectors. Adapter sequences are defined as following:
5' and 3' segment adapters are appended to all segments. Said adapters contain short regions of homology (35-250bp) to the integration site of the destination vector and restriction enzyme recognition sites (ideally of a type IIS restriction enzyme) to permit release of assembled segments form the cloning vector.
5' and 3' block adapters are appended to all blocks. Said adapters contain short regions of homology (15-200bp) to the integration site of the destination vector and restriction enzyme recognition sites (ideally of a type IIS restriction enzyme) to permit release of assembled segments form the cloning vector.
5' and 3' subblock adapters are appended to all subblocks. Said adapters contain short regions of homology (15-100bp) to the integration site of the destination vector and restriction enzyme recognition sites (ideally of a type IIS restriction enzyme) to permit release of assembled segments form the cloning vector.

Adapter sequences are appended to subblocks according to following design rules. If the 5' sequence of a subblock corresponds to the 5' sequences of a segment, a 5' segment adapter is appended to the 5' of said subblock. If the 3' sequence of a subblock corresponds to the 3' sequence of a segment, a 3' segment adapters is appended to the 3' of the said subblock. Furthermore, if the 5' sequence of a subblock corresponds to the 5' sequences of a block, a 5' block adapter is appended to the 5' of the said subblocks. If the 3' sequence of a subblock corresponds to the 3' sequences of a block, a 3' block adapter is appended to the 3' of the said subblocks. Furthermore, to each subblock 5' and 3' subblock adapters are appended to the 5' and 3 termini. When multiple adapter sequences are appended, subblock adapters will be the outermost adapters, followed by block adapters and, where applicable, followed by segment adapters.

In certain embodiments, additional terminal barcode adaptor sequences comprising of a unique barcode sequences are added to both ends of subblocks. Said adaptor sequences contain specific primer annealing sites for subsequent parallel PCR amplification of sets of subblock that serve as assembly units to assemble individual blocks. All subblocks for a given segment contain on one end (5' terminus) identical segment-specific barcode sequences while on the other end (3' terminus) they contain block-specific barcode sequences that facilitate amplification of all subblock for a given block from a library of subblocks (provided upon de novo DNA synthesis).

In certain embodiments, adapter sequences can be omitted if linear dsDNA subblocks are used as building blocks.

*Fifth Step: de novo DNA synthesis of synonymous sequence variants pools -* All DNA subblock variants are synthesized by de novo DNA synthesis yielding a library of double stranded DNA. Each subblocks exists in one or more synonymous sequence variants.

Due to limits in de novo DNA synthesis yield (approx. 80% for 1 kb gene synthesis) not every subblock variant can be successfully generated, however, due to recoding, known or hidden sequence constraints that impede de novo DNA synthesis of a particular subblock variant are not propagated across sequence variants. Increasing the number of sequence variants for which synthesis is attempted will increase the probability that at least one of the synonymous sequence variants can be manufactured.

*Sixth Step: Serial PCR to isolate sets of subblock variants necessary to build each block -* The library of subblocks double stranded DNA variants is used as template for parallel PCR amplification of individual subblock pools. Each PCR amplified subblock pool will contain all successfully synthesized subblock sequence variants needed to build a particular block. Methods for PCR amplification of said subblock pools include current PCR protocols for DNA sequence amplification known in the art and use
- a first primer capable of specifically annealing to the said segment-specific barcode sequences present at terminal regions of subblock variants
- a second primer capable of annealing to the said block specific barcode sequences present at terminal regions of subblock variants.

The skilled artisan understands that amplificates must be discernible by their sequence, i.e. PCR primers must be selected that are placed in such fashion as to allow such distinction.

*Seventh Step: Removal of terminal PCR barcode sequences and higher order assembly of the construct -* Following PCR amplification, terminal barcode sequences attached to individual pools of subblocks are released by restriction endonuclease digest (Bbsl or similar restriction enzymes that recognize 5' and 3' subblock adater sequences). Ensembles of synonymous subblocks are simultaneously (in pooled reactions) assembled into subsequent higher-order assemblies using homologous end joining known in the art and use. Arrays of blocks generated thereby are then released from cloning vectors by restriction enzymes digest (BspQI or similar restriction enzymes that recognize 5' and 3' block adapter sequences) and further assembled into segments. Arrays of segments generated are then released from cloning vectors by restriction enzyme digest (PacI, PmeI or CeuI, SceI or similar restriction enzymes that recognize 5' and 3' segment adapter sequences) and subsequently assembled into the final larger (genome) constructs. As the higher-order assembly does no longer depends on successful synthesis of each DNA subblock variant, large-scale DNA designs can be quickly completed allowing for cost-effective and highly parallelised assembly of extensive genetic part libraries and variants of multi-kilo base long synthetic DNA constructs encoding synthetic pathways or entire synthetic genomes.

The process described herein does not depend on prior knowledge of de novo DNA synthesis feasibility of the DNA units to be manufactured.

In certain embodiments assembly of non-sequence verified synthetic DNA units as well as combinatorial part libraries composed of hundreds to thousands of genetic elements is performed.

Wherever alternatives for single separable features are laid out herein as "embodiments", it is to be understood that such alternatives may be combined freely to form discrete embodiments of the invention disclosed herein.

### Examples

### Description of proof of concept study:

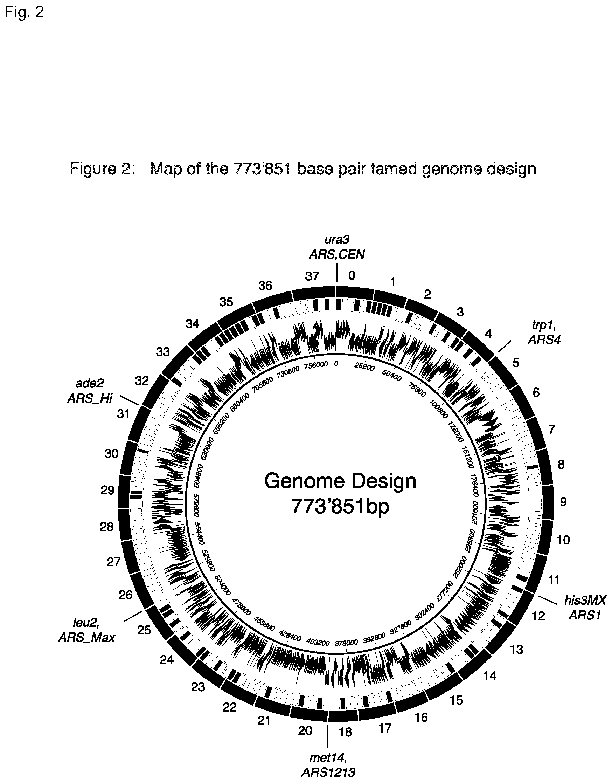

Using hyper-saturated transposon mutagenesis coupled to high throughput sequencing (*Tnseq*)*,* the inventors recently identified the entire set of essential sequences of the cell-cycle model organism *Caulobacter crescentus.* Out of these sequences, the inventors have generated the comprehensive genome-wide list of DNA sequences (DNA parts) encoding the most fundamental functions of a bacterial cell. In particular, parts lists covering all essential and high-fitness functions have been defined for the cell-cycle model organism *Caulobacter crescentus.* The multiplexed DNA part definition approach, including wetlab procedures, bioinformatics pipeline and refactoring of DNA sequences is described in (CHRISTEN, M., et al. http://doi.org/10.1021/acssynbio.5b00087). The part list comprises of 596 single and composite DNA parts encoding essential proteins, RNA and regulatory features. Part boundaries of protein-coding genes have been set to the coding sequence coordinates according to the *Caulobacter* NA1000 genome annotation (NCBI Accession: NC_011916.1) plus additional 5' regulatory sequences (promoters) and terminator region. Boundaries of regulatory upstream sequences were set according to previously identified essential promoter regions (CHRISTEN, B., ABELIUK, E., COLLIER, J. M., KALOGERAKI, V. S., PASSARELLI, B., COLLER, J. A., et al. (2011). The essential genome of a bacterium. Mol. Syst. Biol., 7(1), 528-528. http://doi.org/10.1038/msb.2011.58) and, when necessary, enlarged to include strong transcriptional start sites as determined by RNASeq (BO ZHOU, B., SCHRADER, J. KALOGERAKI, V.S., ABELIUK, E., DINH, C. D., et al. (2015). The global regulatory architecture of transcription during the Caulobacter cell cycle., 11(1), e1004831. http://doi.org/10.1371/journal.pgen.1004831). For essential or high-fitness genes, predicted Rho-independent terminator sequences (GARDNER, P. P., BARQUIST, L., BATEMAN, A., NAWROCKI, E. P., & WEINBERG, Z. (2011). RNIE: genome-wide prediction of bacterial intrinsic terminators. Nucleic Acids Research, 39(14), 5845-5852. http://doi.org/10.1093/nar/gkr168) were included. Essential and high-fitness DNA parts were concatenated in order and orientation as found on the wild-type genome and compiled into a 773'851 base pair long tamed genome design (Figure 2). The genome design implements strong sequence refactoring, part restructuring and complete recoding of all coding sequences. Sequence design and methods of sequence recoding are described in EP15195390.8, hereby incorporated by reference in its entirety.

To locate the most problematic sequences for de novo DNA synthesis, the genome design was partitioned into thirty-seven 20kb long genome segments that were further partitioned into 236 DNA building blocks ordered from a commercial provider of *de novo* DNA synthesis (Gen9, Inc. Cambridge, MA, USA). Out of these, 181 blocks were manufactured by Gen9 Inc. (75.3% success rate) while for 55 blocks de novo DNA synthesis failed (Table 1). This result demonstrates that the current state of the art in de novo DNA synthesis cannot produce every DNA assembly unit with 100% yield using low-cost de novo DNA synthesis methods.

Among the sequence proved to be most difficult to synthesize was segment 25 (21.3 kb in size) for which for 3 out of 6 assembly blocks failed in de novo DNA synthesis (Table 1).

The inventors used the above outlined strategy of multiplexed evolution guided genome assembly to perform neutral recoding of said segment 25 and generate a set of 3 design variants. On average, each design variant contains 2'832 base substitutions corresponding to 13.6% of the sequence replaced with synonymous codon substitutions randomly distributed among the open reading frames (Table 2), excluding immutable regions of THRs and overlapping coding sequences.

Segment 25 was manufactured in three variants by de novo DNA synthesis to yield a library of subblock variants as double stranded DNA. Out of the 60 subblocks ordered from a commercial provider of de novo DNA synthesis (Gen9 Inc), 52 were successfully synthesized, while for 8 subblocks synthesis failed (Table 3 and Figure 3A). As a result, no complete set of subblocks was obtained for any single DNA design illustrating the current shortcomings in de novo DNA synthesis methods for reliable manufacturing of double stranded DNA sequences.

Pools of subblock variants for all five blocks of segment 25 were amplified in 5 PCR reactions (Figure 3B). Each PCR contained a pair of specific PCR primers (Table 6) for amplification the subpool of subblocks necessary for a given block assembly. The PCR-amplified subblocks pools were digested with a type IIS restriction enzyme (Bbsl) to cleave PCR adapter sequences. Each digestion reaction contained a pool of all four sub-blocks to be assembled into a given block, with each subblock represented itself in three design variants. This resulted in a total of five independent digestion reactions for segment 25. The resulting libraries of linear subblock DNA were assembled into their corresponding blocks and integrated into a destination vector (pXMCS-2) using isothermal assembly reactions in a volume of 20 µl. As a control reaction, the inventors performed assembly reactions for block #3 of segment 25 using as templates only subblocks from design variants 1, 2 or 3. None of the individual (incomplete) assembly reactions yielded positive clones for successful assembly of block #3. A PCR pool containing all subblock variants of block #3 yielded an array of correctly assembled blocks each containing synonymous combinations of subblock variants (Table 4). The 4kb DNA blocks were subsequently assembled into 20kb segments and cloned into the low copy plasmid pMR10Y using yeast recombineering (Figure 3E, 3F). The assembled 20kb synthetic segment were sequence verified using standard Sanger sequencing.

Assembly reactions with PCR amplified subpools of subblock variants yielded comparable numbers of colonies compared to control reaction using equimolar rations of individually added subblocks #1-4 (Table 4). Because the serial PCR procedure of the invention amplifies subpools containing all existing synonymous subblock design variants for a given block assembly reaction in a single process step, elaborate pre-analysis of de novo DNA synthesis subblock yield as well as extensive liquid handling steps are not needed.

With redundant DNA synthesis strategies in place to manufacture large-scale DNA sequence, it will become feasible to design and manufacture artifical biosystems in a cost-effective manner. On one hand, this will have fare reaching consequences on how fast functional synthetic genome designs can be accomplished. In addition, greater sequence flexibility enables more dramatic sequence refactoring, including sequence optimization for de novo DNA synthesis, codon usage adaptations, genetic code editing, and recoding of CDSs to erase overlapping gene regulatory features that cause interference between DNA parts and or host cells. Furthermore, de-fragmentation for grouping together related genetic functions to facilitate co-regulation and exchange becomes feasible (for example grouping together tRNAs or genes involved in lipid metabolism, genome replication and stability, etc.).

### Materials and Methods:

### Design of a synthetic essential genome construct.

The comprehensive list of DNA sequences (DNA parts) encoding essential and high-fitness functions required for rich-media growth of *Caulobacter crescentus* was generated using a previously identified essential genome data set (CHRISTEN, B., et al. http://doi.org/10.1038/msb.2011.58) The DNA part list includes DNA sequences encoding proteins, RNA and regulatory features as well as small essential inter-genic sequences. Part boundaries of protein coding genes were set to the CDS coordinates according to the *Caulobacter crescentus* NA1000 genome annotation (NCBI Accession: NC_011916.1) plus additional 5' regulatory sequences (promoters) and terminator regions. Boundaries of regulatory upstream sequences of essential genes were set according to previously identified essential promoter regions and, when necessary, were enlarged to include strong transcriptional start sites as determined by RNAseq. For essential or high-fitness genes, predicted Rho-independent terminator sequences were included. Essential and high-fitness DNA parts were concatenated in order and orientation as found on the wild-type genome and compiled into a 773'354 base pair long synthetic genome constructs. This genome construct was then partitioned into thirty-eight 20 kb long segments (Figure 3)

### Sequence optimization and variant generation of the tamed genome design.

To optimize the sequence of the synthetic genome segments protein-coding sequences were refactored by neutral recoding (synonymous codon replacement) to erase disallowed sequence patterns known to inhibit large-scale de novo DNA synthesis. The average recoding probability across segments was set to 0.57, resulting in introduction of 133354 base substitutions across the 773851 bp genome design. The first four amino acids codons of CDS were excluded from recoding to maintain potential translational and other regulatory signals. Disallowed sequences removed upon recoding included endonuclease sites for BsaI, AarI, BbsI, BspQI, PacI and PmeI, SceI and CeuI. Furthermore, the AGT, ATA, AGA, GTA and AGG codons, which are rare codons in *Caulobacter crescentus,* were set as immutable codons (neither replaced or introduced upon recoding). The amber stop codons TAG and the two TTA and TTG codon for leucine were erased upon recoding. Occurrence of homopolymeric sequences and di and tri-nucleotide repeats were removed (less than six G, eight C', nine A or T, dinucleotides less than 10 repeats, trinucleotides less than 6 repeats). Similarly, direct and indirect sequence repeats larger than 11 bp were removed. To generate variant designs of segment 25, a first recoding of the native sequence design was performed to remove any synthesis constraint. GC and AT content was set to not exceed 70% within a 99bp window and not to exceed 85% within a 21 bp window. To generate subsequent design variants of segment 25, global recoding probability was set to 0.4. For design variant 1, the GC and AT limits were set to 0.62 and 0.8 for a 99bp and 21 bp window size respectively, for design variant 2, the GC and AT limits were set to 0.58 and 0.75 for a 99bp and 21 bp window size respectively and for design variant 3, the GC and AT limits were set to 0.54 and 0.70 for a 99bp and 21 bp window size respectively.

### Parallel PCR-amplification of sub-block pools

Sub-block sequences encompassing design variants of segment 25 were contained in a pG9m-2 low-copy number plasmid library representing all design variants of subblocks form segment 25 that have been successfully manufactured (Table 3 and Figure 3). Sub-pools of subblocks for assemblies of blocks [0-4] were individually amplified using a Phusion® High-Fidelity DNA Polymerase in a 25 µl PCR reaction volume containing: 0.25 µl (2.5 u) Phusion® High-Fidelity DNA Polymerase (New England Biolabs (NEB), USA), 5 µl 5x Phusion® HF Reaction Buffer (NEB), 0.3 µl (∼ 30 ng) plasmid template library of subblock design variants from segment 25, 0.125 µl 100 µM forward primer (block specific barcode), 0.125 µl 100 µM reverse primer (segment barcode primer), 2.5 µl dNTPs (2 mM each) (Thermo Fisher Scientific Inc., USA), 0.75 µl DMSO (Fisher Scientific, UK), and 16 µl ddH20. The PCR was conducted on a BIORAD S1000TMThermal Cycler (Bio-Rad Laboratories Inc., USA) with the following protocol: (1) initial denaturation 3:00 min at 95°C, (2) denaturation 30 s at 95°C, (3) primer annealing 30 s at 58°C, (4) elongation 1:30 min at 72°C, (5) repeat steps 2 - 4 25 times, (6) final elongation 5 min at 72°C.

### Digestion of sub-blocks and pXMCS-2 target vector

The PCR-amplified sub-blocks pools were digested with a Bbsl type IIS restriction enzyme. Each digestion reaction contained a pool of all four sub-blocks variants of a corresponding block resulting in a total of five independent digestion reactions for segment 25. The digestion of each of the five sub-block pools was subsequently performed in a 20 µl reaction volume containing: 10 µl of the sub-block pool directly taken from the PCR reaction mixture, 0.5 µl (5 u) Bbsl type µIIS restriction enzyme (NEB, USA), 2 µl 10x NEBuffer2.1 (NEB, USA), and 7.5 µl nuclease-free H20 (Promega, USA). The digestion reactions were incubated at 37°C overnight and subsequently purified over column and eluted in 20 µl using the NucleoSpin® Gel and PCR clean up Kit (Macherey-Nagel, Switzerland).

The pXMCS-2 target vector was digested with the Ndel and Nhel-HF restriction enzymes in a 40 µl digestion reaction volume composed of: 20 µl (294.4 ng/µl) pXMCS-2, 0.5 µl (10 u) Ndel (NEB, USA), 0.5 µl (10 u) Nhel-HF (NEB, USA), 4 µl 10x CutSmart® buffer (NEB, USA), and 15 µl nuclease-free H20 (Promega, USA). The digestion reaction was incubated at 37°C for 4 h. To verify a successfull digestion, the complete reaction mixture was loaded on a 1% agarose gel (UltraPure™ Agarose, Invitrogen, USA) and run for 40 min at 120 V. The band containing the digested vector was extracted from the gel, purified and eluted in 20 µl using the NucleoSpin® Gel and PCR clean up Kit (Macherey-Nagel, Switzerland). To ensure thorough and complete digestion, the gel-purified digest was re-digested using the same protocol as in the first round digestion, except for an overnight incubation at 37°C and a direct clean-up and purification of the reaction mixture and without the intermediate agarose purification.

### DNA assembly of sub-blocks into blocks:

The Bbsl-digested sub-block pools were assembled into their corresponding blocks and integrated into their target vector pXMCS-2 in a isothermal 20 µl assembly reaction using: 4 µl 5x isothermal reaction buffer, 0.008 µl (0.08 u) T5 Exonuclease (NEB, USA), 0.25 (2.5 u) Phusion® High-Fidelity DNA Polymerase (NEB, USA), 2 µl (80 u) Taq DNA Ligase, 8.742 µl nuclease-free H20 (Promega, USA).

### Electroporation of assembled blocks into E. coli

5 µl of each of the pXMCS-2::block[0-4] assemblies were taken and dialysed on 0.025 µm VSWP MF™ membrane filters (Merck Millipore Ltd., IRL) for 20 min. Following up, the dialysed 5 µl reaction solutions were each electroporated into competent *E. coli* strain DH5α (90 µl aliquots, OD ∼ 15) at 1.75 kV, 400 Q, and 25 µF using 0.1 cm electrode gap Gene Pulser® cuvettes (Bio-Rad Laboratories, USA). The pulse was applied at time constants between 8.6 and 8.8 ms. Immediately after the electroporation, transformed *E. coli* DH5α were rescued in 1 ml SOC medium and incubated at 37°C for 1 h. 100 µl of each rescued electroporation cell sample was plated onto selective LB + kanamycin (20 µg/ml) plates and incubated at 37°C overnight.

### PCR over subblock junctions to verify block assembly

Correct block assemblies were verified using the Genome Partitioner's automatically designed primers sets (Table 7). Subblock junctions were amplified directly by colony PCR from *E. coli* DH5αcontaining pXMCS-2::block[0-4]. Colonies were picked and grown in liquid LB broth supplemented with kanamycin (20 µg/ml). PCR amplification of subblock junctions for each block was performed using the liquid culture as template. In 20 µl final reaction volume 10 µl 2x GoTaq® G2 Green Master Mix (Promega, USA), 0.5 µl 100 µM forward primer (fw primers of #3-32), 0.5 µl 100 µM reverse primer (rv primers of #3-32), 1 µl DH5α pXMCS-2::block[0-4] liquid culture, and 8 µl ddH2O were added. The PCR protocol consisted of: (1) initial denaturation 3:00 min at 95°C, (2) denaturation 30 s at 95°C, (3) primer annealing 30 s at 60°C, (4) elongation 30 s min at 72°C, (5) repeat steps 2 - 4, 25 times, (6) final elongation 5 min at 72°C.

### BspQI-mediated Block release from pXMCS-2 vector

Plasmids pXMCS-2::block[0-4] were purified from the respective DH5αstrain (see strains, BC3744-BC3748, Table 8) using the GeneJET Plasmid Miniprep Kit (Thermo Scientific, USA). Subsequently, the blocks were released from the pXMCS-2 backbone via a BspQI type IIS restriction digestion (Figure 3C). Each block release consisted of a 40 µl digestion reaction volume composed of: 10 µl (> 5 µg) pXMCS-2::block[0-4] plasmid, 1µl (10 u) BspQI type IIS restriction enzyme (NEB, USA), 4 µl 10x NEBuffer 3.1 (NEB, USA), and 25 µl nuclease-free H2O (Promega, USA). The digestions were incubated at 50°C for 1.5 h and in the following the reactions stopped via an incubation at 80°C for 20 min. Digested constructs were columns purified using the NucleoSpin® Gel and PCR clean up Kit (Macherey-Nagel, Switzerland).

### Yeast assembly of segment from blocks[0-4]

Column-purified blocks[0-4] were used for assembly of segment 25 into a pMR10Y (pMR10::*CEN*/*ARS::ura3*) plasmid backbone. *S*. *cerevisiae* strain VL6-48N (BC3347) was grown until OD600 0.7 of which 2 ml were pelleted and then resuspended in 1 ml 0.9% NaCl-solution. The culture was pelleted again, the NaCl-solution supernatant discarded and 100 µg fish sperm DNA added (single stranded from salmon testes, D7656, Sigma-Aldrich, USA). Subsequently, ∼ 540 µg linearized pMR10Y and ∼ 300 µg of each block digest was added to the pellet. After thorough vortexing the pellet was resuspended in 500 µl transformation mixture (400 µl 50% PEG solution, 50 µl 1M Lithium acetate, 50 µl ddH2O). To complete the transformation, 57 µl DMSO were added to the transformation reaction and incubated at RT for 15 min, followed directly by a heat-shock incubation of 15 min at 42°. Finally, the culture was pelleted, the supernatant discarded, the pellet was resuspended in 100 µl ddH2O and plated onto a yeast synthetic drop-out medium (w/o uracil, + glucose (10 g/L), + adenine (80 mg/L) and incubated at 30°C for three days.

### Yeast colony PCR to verify segment 25 block junctions

Using the Genome Partitioner's automatically designed primers sets the correct segment assembly was verified by amplifying each block junction directly by PCR on transformed yeast colonies from the assembly step above. Six colonies were picked and grown in liquid yeast synthetic drop-out medium (w/o uracil, + glucose (10 g/L), + adenine (80 mg/L). The PCR to amplify the block junctions was performed in a 20 µl reaction volume as follows: 10 µl 2x Phire Green Hot Start II PCR Master Mix (Thermo Scientific, USA), 0.5 µl 25 µM forward primer (fw primers of #33-40), 0.5 µl 25 µM reverse primer (rv primers of #33-40), 1 transformed yeast liquid culture, and 8 µl ddH2O. The PCR protocol consisted of: (1) initial denaturation 3:00 min at 98°C, (2) denaturation 5 s at 98°C, (3) primer annealing 5 s at 62°C, (4) elongation 20 s min at 72°C, (5) repeat steps 2 - 4, 40 times, (6) final elongation 1 min at 72°C

### Partitioning parameters, DNA adapter sequences and barcodes used.

The following partitioning parameters were applied: Segment size: 20'000 bp, Segment overlap: 120 bp, Block size: 4'000 bp, Block overlap: 80bp, Subblock size 1'000 bp, Subblock overlap: 25 bb. Adaptor sequences used for partitioning are listed in table 5, barcode primers used for subpool PCR amplification are listed in table 6. Primers used for PCR verification of block assembly are listed in Table 7.

**Table 1: de novo DNA synthesis yield of tamed genome partitioned as 4 kb blocks**

| Segment | Coordinates, size [bp] | Blocks | Synthesis failed | Yield [%] |
|---|---|---|---|---|
| 0 | 1..22276 [22275 bp] | 6 | block[0], block[3], block[5] | 50.0 |
| 1 | 22157..41386 [19229 bp] | 6 | block[0], block[1], block[2], block[3] | 33.3 |
| 2 | 41267..60570 [19303 bp] | 6 | block[1] | 83.4 |
| 3 | 60451..80086 [19635 bp] | 6 | block[0], block[3], block[5] | 50.0 |
| 4 | 79968..101065 [21097 bp] | 6 | block[0], block[2], block[4] | 50.0 |
| 5 | 100946..122063 [21117 bp] | 6 | | 100.0 |
| 6 | 121944..142293 [20349 bp] | 8 | | 100.0 |
| 7 | 142174..161366 [19192 bp] | 7 | | 100.0 |
| 8 | 161247..182490 [21243 bp] | 8 | block[3] | 87.5 |
| 9 | 182371..202617 [20246 bp] | 8 | | 100.0 |
| 10 | 202498..223202 [20704 bp] | 8 | | 100.0 |
| 11 | 223083..245967 [22884 bp] | 6 | block[4] | 83.3 |
| 12 | 245848..266862 [21014 bp] | 6 | block[0], block[5] | 67.2 |
| 13 | 266762..288128 [21366 bp] | 6 | block[1] | 83.3 |
| 14 | 288009..309976 [21967 bp] | 6 | block[1], block[2] | 66.7 |
| 15 | 309857..332605 [22748 bp] | 6 | block[0] | 83.3 |
| 16 | 332486..351748 [19262 bp] | 6 | | 100.0 |
| 17 | 351627..374062 [22435 bp] | 6 | block[0], block[4] | 66.7 |
| 18 | 373943..391434 [17491 bp] | 5 | block[2] | 80.0 |
| 20 | 391316..413535 [22219 bp] | 6 | block[1], block[4] | 66.7 |
| 21 | 413414..434554 [21140 bp] | 6 | block[4] | 83.3 |
| 22 | 434433..456204 [21771 bp] | 6 | block[4], block[5] | 66.7 |
| 23 | 456085..476452 [20367 bp] | 6 | block[2], block[5] | 66.7 |
| 24 | 476332..496786 [20454 bp] | 6 | block[0], block[4] | 66.7 |
| 25 | 496667..518079 [21412 bp] | 6 | block[1block[3], block[4] | 50.0 |
| 26 | 517978..539585 [21607 bp] | 6 | | 100.0 |
| 27 | 539466..559225 [19759 bp] | 6 | | 100.0 |
| 28 | 559106..577887 [18781 bp] | 7 | | 100.0 |
| 29 | 577768..597047 [19279 bp] | 7 | block[2], block[3] | 71.2 |
| 30 | 596928..617171 [20243 bp] | 8 | block[6] | 87.3 |
| 31 | 617052..638739 [21687 bp] | 6 | | 100.0 |
| 32 | 638620..659187 [20567 bp] | 6 | | 100.0 |
| 33 | 659068..681645 [22577 bp] | 6 | block[0], block[5] | 66.7 |
| 34 | 681526..702397 [20871 bp] | 6 | block[0], block[1], block[4], block[5] | 33.3 |
| | | | block[0], block[1], block[2], block[3], | |
| 35 | 702278..725151 [22873 bp] | 6 | block[5] | 16.7 |
| 36 | 725032..748643 [23611 bp] | 7 | block[0], block[3] | 71.5 |
| 37 | 748524..773851 [25327 bp] | 7 | block[3], block[5] | 71.4 |
| | **Total size: 778'102 bp** | **236** | **synthesis failed: 55** | **75.4** |

Table 1: The table headers have the following meaning: *Segment:* Segments number as annotated in the tamed genome design, *Coordinates:* Base pair sequence coordinates according to the GenBank file of the genome design, *Size in [bp]:* Length of the Segments in base pairs, *Blocks:* Number of partition blocks used per segment, *Synthesis failed:* list of blocks for which synthesis failed during the first round of de novo DNA synthesis, *Yield [%]:* Percentage of the segment sequence for which de novo DNA synthesis was successful.

**Table 2: Base substitution rates between subblock variant designs of segment 25**

| **SB** | **Coordinates** | | | **Base substitutions rates** | | |
|---|---|---|---|---|---|---|
| **ID** | **Begin** | **End** | **Size [bp]** | **design 1 vs design 2** | **design 1 vs design 3** | **design 2 vs design 3** |
| 0 | 1 | 942 | 942 | 144 (15.3%) | 150 (15.9%) | 155 (16.5%) |
| 1 | 919 | 1972 | 1054 | 95 (9%) | 108 (10.2%) | 115 (10.9%) |
| 2 | 1949 | 3004 | 1056 | 155 (14.7%) | 172 (16.3%) | 182 (17.2%) |
| 3 | 2981 | 4006 | 1026 | 158 (15.4%) | 174 (17%) | 170 (16.6%) |
| 4 | 3926 | 4968 | 1043 | 133 (12.8%) | 130 (12.5%) | 147 (14.1%) |
| 5 | 4942 | 6018 | 1077 | 148 (13.7%) | 153 (14.2%) | 167 (15.5%) |
| 6 | 5995 | 7070 | 1076 | 154 (14.3%) | 168 (15.6%) | 170 (15.8%) |
| 7 | 7046 | 8089 | 1044 | 62 (5.9%) | 68 (6.5%) | 75 (7.2%) |
| 8 | 8009 | 9040 | 1032 | 147 (14.2%) | 157 (15.2%) | 162 (15.7%) |
| 9 | 9017 | 10080 | 1064 | 147 (13.8%) | 169 (15.9%) | 192 (18%) |
| 10 | 10057 | 11123 | 1067 | 163 (15.3%) | 177 (16.6%) | 183 (17.2%) |
| 11 | 11097 | 12134 | 1038 | 154 (14.8%) | 191 (18.4%) | 161 (15.5%) |
| 12 | 12056 | 13088 | 1033 | 120 (11.6%) | 126 (12.2%) | 135 (13.1%) |
| 13 | 13064 | 14127 | 1064 | 144 (13.5%) | 148 (13.9%) | 168 (15.8%) |
| 14 | 14104 | 15170 | 1067 | 158 (14.8%) | 181 (17%) | 209 (19.6%) |
| 15 | 15144 | 16180 | 1037 | 45 (4.3%) | 52 (5%) | 40 (3.9%) |
| 16 | 16100 | 17132 | 1033 | 132 (12.8%) | 120 (11.6%) | 125 (12.1%) |
| 17 | 17109 | 18175 | 1067 | 167 (15.7%) | 184 (17.2%) | 171 (16%) |
| 18 | 18149 | 19217 | 1069 | 130 (12.2%) | 143 (13.4%) | 148 (13.8%) |
| 19 | 19193 | 20148 | 956 | 85 (8.9%) | 99 (10.4%) | 111 (11.6%) |

Table 2: The table headers have the following meaning: *SB ID*: Sublock number as annotated in the tamed genome design, *Coordinates:* Base pair sequence coordinates according to the GenBank file of the genome design, *Size in [bp]:* length of the Segments in base pairs, *Base substitution rates:* Number of base substitutions of subblocks occurring between design variants, *Begin:* Genome coordinates of subblock start position, *End:* Genome coordinates of subblock end position, *Size [bp]:* Size of subblock in base pairs.

**Table 3: De novo DNA synthesis yield of 3 subblock design variants from segment 25**

| **Design** | **Block** | **Subblock** | **Length** | **Yield (ng)** | **Vector** | **Strain ID** |
|---|---|---|---|---|---|---|
| 1 | 0 | 0 | 1071 bp | 655 | pG9m-2 | BC3682 |
| 1 | 0 | 1 | 1070 bp | 475 | pG9m-2 | BC3683 |
| 1 | 0 | 2 | 1072 bp | 655 | pG9m-2 | BC3684 |
| 1 | 0 | 3 | 1074 bp | 515 | pG9m-2 | BC3685 |
| 1 | 1 | 4 | 1092 bp | 525 | pG9m-2 | BC3686 |
| 1 | 1 | 5 | 1093 bp | 450 | pG9m-2 | BC3687 |
| 1 | 1 | 6 | 1092 bp | 481.5 | pG9m-2 | BC3688 |
| 1 | 1 | 7 | 1092 bp | 494 | pG9m-2 | BC3689 |
| 1 | 2 | 8 | 1081 bp | failed in DNA | synthesis | |
| 1 | 2 | 9 | 1080 bp | 515 | pG9m-2 | BC3690 |
| 1 | 2 | 10 | 1083 bp | 387.5 | pG9m-2 | BC3691 |
| 1 | 2 | 11 | 1086 bp | 550 | pG9m-2 | BC3692 |
| 1 | 3 | 12 | 1082 bp | failed in DNA | synthesis | |
| 1 | 3 | 13 | 1080 bp | 570 | pG9m-2 | BC3693 |
| 1 | 3 | 14 | 1083 bp | 362.5 | pG9m-2 | BC3694 |
| 1 | 3 | 15 | 1085 bp | 555 | pG9m-2 | BC3695 |
| 1 | 4 | 16 | 1082 bp | 369 | pG9m-2 | BC3696 |
| 1 | 4 | 17 | 1083 bp | 406.5 | pG9m-2 | BC3697 |
| 1 | 4 | 18 | 1085 bp | 550 | pG9m-2 | BC3698 |
| 1 | 4 | 19 | 1084 bp | 394 | pG9m-2 | BC3699 |
| 2 | 0 | 0 | 1071 bp | 375 | pG9m-2 | BC3648 |
| 2 | 0 | 1 | 1070 bp | 394 | pG9m-2 | BC3650 |
| 2 | 0 | 2 | 1072 bp | 331.5 | pG9m-2 | BC3651 |
| 2 | 0 | 3 | 1074 bp | 615 | pG9m-2 | BC3653 |
| 2 | 1 | 4 | 1092 bp | 481.5 | pG9m-2 | BC3655 |
| 2 | 1 | 5 | 1093 bp | failed in DNA | synthesis | |
| 2 | 1 | 6 | 1092 bp | 406.5 | pG9m-2 | BC3657 |
| 2 | 1 | 7 | 1092 bp | 690 | pG9m-2 | BC3658 |
| 2 | 2 | 8 | 1081 bp | 375 | pG9m-2 | BC3660 |
| 2 | 2 | 9 | 1080 bp | 755 | pG9m-2 | BC3662 |
| 2 | 2 | 10 | 1083 bp | 469 | pG9m-2 | BC3664 |
| 2 | 2 | 11 | 1086 bp | 306.5 | pG9m-2 | BC3666 |
| 2 | 3 | 12 | 1082 bp | failed in DNA | synthesis | |
| 2 | 3 | 13 | 1080 bp | failed in DNA | synthesis | |
| 2 | 3 | 14 | 1083 bp | 469 | pG9m-2 | BC3669 |
| 2 | 3 | 15 | 1085 bp | 381.5 | pG9m-2 | BC3671 |
| 2 | 4 | 16 | 1082 bp | 350 | pG9m-2 | BC3672 |
| 2 | 4 | 17 | 1083 bp | 469 | pG9m-2 | BC3673 |
| 2 | 4 | 18 | 1085 bp | 640 | pG9m-2 | BC3675 |
| 2 | 4 | 19 | 1084 bp | 331.5 | pG9m-2 | BC3677 |
| 3 | 0 | 0 | 1071 bp | 362.5 | pG9m-2 | BC3649 |
| 3 | 0 | 1 | 1070 bp | 331.5 | pG9m-2 | BC3652 |
| 3 | 0 | 2 | 1072 bp | 615 | pG9m-2 | BC3654 |
| 3 | 0 | 3 | 1074 bp | 337.5 | pG9m-2 | BC3656 |
| 3 | 1 | 4 | 1092 bp | failed in DNA | synthesis | |
| 3 | 1 | 5 | 1093 bp | 375 | pG9m-2 | BC3659 |
| 3 | 1 | 6 | 1092 bp | 795 | pG9m-2 | BC3661 |
| 3 | 1 | 7 | 1092 bp | 325 | pG9m-2 | BC3663 |
| 3 | 2 | 8 | 1081 bp | 1065 | pG9m-2 | BC3665 |
| 3 | 2 | 9 | 1080 bp | failed in DNA | synthesis | |
| 3 | 2 | 10 | 1083 bp | 331.5 | pG9m-2 | BC3667 |
| 3 | 2 | 11 | 1086 bp | 690 | pG9m-2 | BC3668 |
| 3 | 3 | 12 | 1082 bp | 505 | pG9m-2 | BC3670 |
| 3 | 3 | 13 | 1080 bp | failed in DNA | synthesis | |
| 3 | 3 | 14 | 1083 bp | 900 | pG9m-2 | BC3674 |
| 3 | 3 | 15 | 1085 bp | 319 | pG9m-2 | BC3676 |
| 3 | 4 | 16 | 1082 bp | 720 | pG9m-2 | BC3678 |
| 3 | 4 | 17 | 1083 bp | 820 | pG9m-2 | BC3679 |
| 3 | 4 | 18 | 1085 bp | 306.5 | pG9m-2 | BC3680 |
| 3 | 4 | 19 | 1084 bp | 312.5 | pG9m-2 | BC3681 |

Table 3: De novo DNA synthesis failed for 8 out of 60 subblocks that build segment 25 in 3 synonymous design variants. None of the design variants yielded all subblocks needed for successful assembly of segment 25. The table headers have the following meaning: *Design:* Sequence design variant, *Block:* Block number, *Subblock:* Subblock number, *Length:* size of subblock generated by de novo DNA synthesis, *Yield (ng):* Yield of plasmid-cloned subblock in nano-gram of DNA, *Strain ID:* Strain identification number.

**Table 4: Efficiency of block assembly reactions using pools of subblock variants**

| Assembly reaction | Subblock design variants | | | | Number of |
|---|---|---|---|---|---|
| | sb 0 | sb1 | sb2 | sb3 | colonies^{a} |
| *Assembly reactions with PCR amplified subpools of subblock variants:* | | | | | |
| Block_0_all | 1,2,3 | 1,2,3 | 1,2,3 | 1,2,3 | 41 |
| Block_1_all | 1,2 | 1,3 | 1,2,3 | 1,2,3 | 31 |
| Block_2_all | 2,3 | 1,2 | 1,2,3 | 1,2,3 | 88 |
| Block_3_all | 3 | 1 | 1,2,3 | 1,2,3 | 179 |
| Block_4_all | 1,2,3 | 1,2,3 | 1,2,3 | 1,2,3 | 264 |
| *Assembly reactions, block_3 with individual design variants:* | | | | | |
| Block_3_d1 | - | 1 | 1 | 1 | 3 |
| Block_3_d2 | - | - | 2 | 2 | 5 |
| Block_3_d3 | 3 | - | 3 | - | 13 |
| *Assembly reactions using equimolar ratio of subblocks:* | | | | | |
| Block_0 | 1 | 1 | 1 | 1 | 244 |
| Block_1 | 1 | 1 | 1 | 1 | 3^{b} |
| Block_2 | 3 | 1 | 1 | 1 | 155 |
| Block_3 | 3 | 1 | 1 | 1 | 156 |
| Block_4 | 1 | 1 | 1 | 1 | 63 |
| *Assembly reactions using non-equimolar ratios of subblocks* | | | | | |
| Block_3 | 3 | 1 | 1,2,3 | 1,2,3 | 231 |

Table 4: The table headers have the following meaning: *Assembly reaction:* Name of the assembly reaction, *Subblock design variants:* Design variant(s) of a particular subblock that were used during assembly reaction. SB: Subblock number, *Number of colonies:* Colonies obtained after electroporation and outgrowth of corresponding DH5α pXMCS-2::block[0-4] assemblies, ^{a} Controls reactions of only the digested subblocks and the digested pXMCS-2 into *E. coli* DH5α resulted in 0 and 8 colonies, respectively, ^{b} 2 out of the 3 clones of block 1 were confirmed by PCR.

**Table 5: List of adaptor sequences used for partitioning**

| Adapter | Sequence |
|---|---|
| 5' segment adapter | CGGATTTCAATAGCTGATATAGCGAATCACCGAGATTAATTAA |
| 3' segment adapter | GTTTAAACGATACTAGATGTATAATGTCCGCCATGCAGACGAA |
| 5' block adapter | CGAGTTTTGGGGAGACGACCATATGGCTCTTCA |
| 3' block adapter | CGAGTTTTGGGGAGACGACCATATGGCTCTTCA |
| 5' subblock adapter | GAAGACAA |
| 3' subblock adapter | TTGTCTTC |

Table 5: *Adapter:* Type of adapter, *Sequence:* Adaptor DNA sequence.

**Table 6: List of barcode primers used subpool PCR amplification**

| Barcode# | Primer | Sequence |
|---|---|---|
| 1 | 5'-barcode1_blc_0 | GCGTTCGCTCTAAGAGTC |
| 2 | 5'-bar2_blc_1 | AGTCGTCTCATCGGTAGC |
| 3 | 5'-bar3_blc_2 | GGCTGATACTCGCTACGT |
| 4 | 5'-bar4_blc_3 | GCCGTCGGTAGTTCATAC |
| 5 | 5'-bar5_blc_4 | CTTTCCCTAGACGGAGGT |
| 6 | 3'-bar6_segm25 | CGTCCGGTTGAAGTCTAC |

Table 6: *Barcode* #: Barcode ID, *Primer:* Name of the primer, *Sequence:* DNA sequence of the oligonucleotide primer.

**Table 7: List of primers used for PCR verification of block assembly**

| Primer # | Primer ID | Junction | Sequence |
|---|---|---|---|
| 1 | BC1484 | pG9m2_cloningsite_fw | GTGAAGGTGAGCCAGTGA |
| 2 | BC1485 | pG9m2_cloningsite_rv | GAAAGTCAAAAGCCTCCG |
| 3 | A1 | >subbl_ov_0_1_fw | CCTGCACAGGCTCGACGATG |
| 4 | A2 | >subbl_ov_0_1_rv | CGTTCGCCGACGTGGTGTTC |
| 5 | A3 | >subbl_ov_1_2_fw | GCCAAGCAACTAGGCGGCGT |
| 6 | A4 | >subbl_ov_1_2_rv | GCGACGACCGCAGAAGGTGA |
| 7 | A5 | >subbl_ov_2_3_fw | CCTGTCAGGTGCTGGTCTGG |
| 8 | A6 | >subbl_ov_2_3_rv | GGCGATCCGAGACGAAGTCG |
| 9 | A7 | >subbl_ov_4_5_fw | CCACACCCATCATGCGCACG |
| 10 | A8 | >subbl_ov_4_5_rv | TCCGCTGGTGATCGACCTGG |
| 11 | A9 | >subbl_ov_5_6_fw | CGCGTGCTATAGGCGAGCCA |
| 12 | A10 | >subbl_ov_5_6_rv | GCGCATCGGCTTCTACAGCG |
| 13 | A11 | >subbl_ov_6_7_fw | ACGCACGCTCCCCTGACCAT |
| 14 | A12 | >subbl_ov_6_7_rv | GGCTCTGCGCTGTTGAGGTC |
| 15 | B1 | >subbl_ov_8_9_fw | GCCATAGCTGCCCCAAGAGC |
| 16 | B2 | >subbl_ov_8_9_rv | GTCGTGCTTTGGGGCGTACG |
| 17 | B3 | >subbl_ov_9_10_fw | CTCCGGAACGGTCGCTTGGA |
| 18 | B4 | >subbl_ov_9_10_rv | TGGTTGTCACCGACGGCGGT |
| 19 | B5 | >subbl_ov_10_11_fw | CGGCGCCGATATTGGCCTTC |
| 20 | B6 | >subbl_ov_10_11_rv | CGGCGCGGTTGTCGAACAGT |
| 21 | B7 | >subbl_ov_12_13_fw | CTCTCGCGGATCGGTCCCTT |
| 22 | B8 | >subbl_ov_12_13_rv | TCGACTCCGGGGCGTTTTCC |
| 23 | B9 | >subbl_ov_13_14_fw | ACCCTTCTTGCGACGTGGGC |
| 24 | B10 | >subbl_ov_13_14_rv | TCGAAGTGAACCTGCCGCCG |
| 25 | B11 | >subbl_ov_14_15_fw | GCTTGTTGAGCGCGGCGAAC |
| 26 | B12 | >subbl_ov_14_15_rv | TTTTGCCCAGGACGCCGCAG |
| 27 | C1 | >subbl_ov_16_17_fw | CAGATAGCCGCGAGCGTACG |
| 28 | C2 | >subbl_ov_16_17_rv | GCGATGTGACCAGCGTCCAG |
| 29 | C3 | >subbl_ov_17_18_fw | TCGATGTCGACGGCGGTCAG |
| 30 | C4 | >subbl_ov_17_18_rv | ATCCACAACGCCGCCTGCGA |
| 31 | C5 | >subbl_ov_18_19_fw | TCAGCATGATCCGGGCGTGC |
| 32 | C6 | >subbl_ov_18_19_rv | GTCGGTCGCAGGATGACGCT |
| 33 | D1 | >block_ov_0_1_fw | GACGCGGTTATCGATGGCGA |
| 34 | D2 | >blockl_ov_0_1_rv | GGTTTCGGGCGGTTGTCCAT |
| 35 | D3 | >b!ock_ov_1_2_fw | AGCAGCATGGCGGGGAAGTT |
| 36 | D4 | >blockl_ov_1_2_rv | CCACCTACAGCTGCTTGCCA |
| 37 | D5 | >block_ov_2_3_fw | CCCACCACGACAATGATGCG |
| 38 | D6 | >blockl_ov_2_3_rv | CCACAAGATCTGGCGCGGTA |
| 39 | D7 | >block_ov_3_4_fw | ACTGAGCTACCCAGGCATCC |
| 40 | D8 | >blockl_ov_3_4_rv | TCGAGACGAAGGTCGGCTTC |

Table 7: *Primer #:* Primer number, *Primer ID:* Name of the primer, *Junction:* Name of the subblock junction, *Sequence:* Primer DNA sequence.

**Table 8: List of strains:**

| **Strain** | **Description** | **Reference or source** |
|---|---|---|
| *Strains harboring 1 kb subblocks in pG9m-2:* | | |
| BC3648 | *E. coli* (DH5α), pG9m-2::d2_blc:0_4151,sb:0_1071 | this work |
| BC3649 | *E. coli* (DH5α), pG9m-2::d3_blc:0_4151,sb:0_1071 | this work |
| BC3650 | *E. coli* (DH5α), pG9m-2::d2_blc:0_4151,sb:1_1070 | this work |
| BC3651 | *E. coli* (DH5α), pG9m-2::d2 blc:0 4151,sb:2_1072 | this work |
| BC3652 | *E. coli* (DH5α), pG9m-2::d3_blc:0_4151,sb:1_1070 | this work |
| BC3653 | *E. coli* (DH5α), pG9m-2::d2_blc:0_4151,sb:3_1074 | this work |
| BC3654 | *E. coli* (DH5α), pG9m-2::d3_blc:0_ 4151,sb:2_1072 | this work |
| BC3655 | *E. coli* (DH5α), pG9m-2::d2_blc:1_4229,sb:4_1092 | this work |
| BC3656 | *E. coli* (DH5α), pG9m-2::d3_blc:0_4151,sb:3_1074 | this work |
| BC3657 | *E. coli* (DH5α), pG9m-2::d2_blc:1_4229,sb:6_1092 | this work |
| BC3658 | *E. coli* (DH5α), pG9m-2::d2_blc:1_4229,sb:7_1092 | this work |
| BC3659 | *E. coli* (DH5α), pG9m-2::d3_blc:1_4229,sb:5_1093 | this work |
| BC3660 | *E. coli* (DH5α), pG9m-2::d2_blc:2_4191,sb:8_1081 | this work |
| BC3661 | *E. coli* (DH5α), pG9m-2::d3_blc:1_4229,sb:6_1092 | this work |
| BC3662 | *E. coli* (DH5α), pG9m-2::d2_blc:2 4191,sb:9_1080 | this work |
| BC3663 | *E. coli* (DH5α), pG9m-2::d3_blc:1_4229,sb:7_1092 | this work |
| BC3664 | *E. coli* (DH5α), pG9m-2::d2_blc:2_4191,sb:10_1083 | this work |
| BC3665 | *E. coli* (DH5α), pG9m-2::d3_blc:2_4191,sb:8_1081 | this work |
| BC3666 | *E. coli* (DH5α), pG9m-2::d2_blc:2_4191,sb:11_1086 | this work |
| BC3667 | *E*. *coli* (DH5α), pG9m-2::d3_blc:2_4191,sb:10_1083 | this work |
| BC3668 | *E*. *coli* (DH5α), pG9m-2::d3_blc:2_4191,sb:11_1086 | this work |
| BC3669 | *E*. *coli* (DH5α), pG9m-2::d2_blc:3- 4190,sb:14_1083 | this work |
| BC3670 | *E*. *coli* (DH5α), pG9m-2::d3_blc:3_4190,sb:12_1082 | this work |
| BC3671 | *E*. *coli* (DH5α), pG9m-2::d2_blc:3_4190,sb:15_1085 | this work |
| BC3672 | *E*. *coli* (DH5α), pG9m-2::d2_blc:4_4194,sb:16_1082 | this work |
| BC3673 | *E*. *coli* (DH5α), pG9m-2::d2_blc:4_4194,sb:17_1083 | this work |
| BC3674 | *E*. *coli* (DH5α), pG9m-2::d3_blc:3_4190,sb:14_1083 | this work |
| BC3675 | *E*. *coli* (DH5a), pG9m-2::d2_blc:4_4194,sb:18_1085 | this work |
| BC3676 | *E*. *coli* (DH5α), pG9m-2::d3_blc:3_4190,sb:15_1085 | this work |
| BC3677 | *E*. *coli* (DH5α), pG9m-2::d2_blc:4_4194,sb:19_1084 | this work |
| BC3678 | *E*. *coli* (DH5α), pG9m-2::d3_blc:4_4194,sb:16_1082 | this work |
| BC3679 | *E*. *coli* (DH5α), pG9m-2::d3_blc:4_4194,sb:17_1083 | this work |
| BC3680 | *E. coli* (DH5α), pG9m-2::d3_blc:4_4194,sb:18_1085 | this work |
| BC3681 | *E*. *coli* (DH5α), pG9m-2::d3_blc:4_4194,sb:19_1084 | this work |
| BC3682 | *E*. *coli* (DH5α), pG9m-2::d1_blc:0_4151,sb:0_1071 | this work |
| BC3683 | *E. coli* (DH5α), pG9m-2::d1_blc:0_4151,sb:1_1070 | this work |
| BC3684 | *E. coli* (DH5α), pG9m-2::d1_blc:0_4151_sb:2_1072, | this work |
| BC3685 | *E. coli* (DH5α), pG9m-2::d1_blc:0_4151,sb:3_1074 | this work |
| BC3686 | *E. coli* (DH5α), pG9m-2::d1_blc:1_4229,sb:4_1092 | this work |
| BC3687 | *E. coli* (DH5α), pG9m-2::d1_blc:1_4229,sb:5_1093, | this work |
| BC3688 | *E. coli* (DH5α), pG9m-2::d1_blc:1_4229,sb:6_1076 | this work |
| BC3689 | *E. coli* (DH5α), pG9m-2::d1_blc:1_4229,sb:7_1092, | this work |
| BC3690 | *E. coli* (DH5α), pG9m-2::d1_blc:2_4191,sb:9_1080 | this work |
| BC3691 | *E. coli* (DH5α), pG9m-2::d1_blc:2_4191,sb:10_1083 | this work |
| BC3692 | *E. coli* (DH5α), pG9m-2::d1_blc:2_4191,sb:11_1086 | this work |
| BC3693 | *E*. *coli* (DH5α), pG9m-2::d1_blc:3_4190,sb:13_1080 | this work |
| BC3694 | *E*. *coli* (DH5α), pG9m-2::d1_blc:3_4190,sb:14_1083 | this work |
| BC3695 | *E*. *coli* (DH5α), pG9m-2::d1_blc:3_4190,sb:15_1085 | this work |
| BC3696 | *E*. *coli* (DH5α), pG9m-2::d1_blc:4_4194,sb:16_1082 | this work |
| BC3697 | *E. coli* (DH5α), pG9m-2::d1_blc:4_4194,sb:17_1083 | this work |
| BC3698 | *E*. *coli* (DH5α), pG9m-2::d1_blc:4_4194,sb:18_1085 | this work |
| BC3699 | *E*. *coli* (DH5α), pG9m-2::d1_blc:4_4194,sb:19_1084 | this work |
| *Strains containing 4 kb DNA blocks of segment 25 in pXMCS-2:* | | |
| BC3744 | *E. coli* (DH5α), pXMCS-2::block0 | this work |
| BC3745 | *E. coli* (DH5α), pXMCS-2::block1 | this work |
| BC3746 | *E. coli* (DH5α), pXMCS-2::block2 | this work |
| BC3747 | *E. coli* (DH5α), pXMCS-2::block3 | this work |
| BC3748 | *E. coli* (DH5α), pXMCS-2::block4 | this work |
| Stain containing synthetic DNA segments in pMR10Y (pMR10::*CEN*/*ARS*::ura3): | | |
| BC3762 | *S*. *cerevisiae* (VL6-48N), pMR10Y::Seg25 | this work |
| Strains used for plasmid cloning and propagation containing | | |
| DH5α | *E*. *coli* (DH5α), electro-competent | this work |
| BC3347 | *S*. *cerevisiae* (VL6-48N) | Larinov et al * |
| | MAT α, his3-D200, trp1-Δ1, ura3-Δ1, lys2, ade2-101, met14, psi+cir° | |

Table headers have the following meaning: Strain: Name of the strain, Description: description of strain and genotype, * LARIONOV, V., KOUPRINA, N., NIKOLAISHVILI, N., & RESNICK, M. A. (1994). Recombination during transformation as a source of chimeric mammalian artificial chromosomes in yeast (YACs). Nucleic Acids Research, 22(20), 4154-4162.

## Claims

1. A process for manufacturing a DNA construct of interest, comprising the steps of
- providing a template *in silico* DNA construct comprising a plurality of genetic elements;
- subjecting said template *in silico* DNA construct to a computational optimization step, wherein one or more sequences inhibiting de novo DNA synthesis are removed from said template *in silico* DNA construct by neutral sequence change, yielding an optimized *in silico* DNA construct, provided that start codons are not removed or replaced;
- partitioning said optimized *in silico* DNA construct into a plurality of original *in silico* assembly units in a partitioning step, wherein said optimized *in silico* DNA construct is partitioned such that in each case two adjacent members of said plurality of original *in silico* assembly units share a terminal homology region, wherein one terminal homology region differs from any other;
- subjecting each member of said plurality of original *in silico* assembly units to a computational synonymous sequence recoding step, wherein
• one or more synonymous *in silico* assembly units are generated for each member of said plurality of original *in silico* assembly units by neutral sequence change, provided that no terminal homology region or start codon is altered, and
• an *in silico* assembly variant pool comprising said member of said plurality of original *in silico* assembly units and said one or more synonymous *in silico* assembly units is generated, thereby yielding a library of *in silico* variant pools;
- de novo synthesizing one or more members of each *in silico* assembly variant pool of said library of *in silico* variant pools, thereby yielding a library of nucleic acid assembly units;
- amplifying said library of nucleic acid assembly units in an amplification step, yielding an amplified library of nucleic acid assembly units; and
- assembling said amplified library of nucleic acid assembly units into said DNA construct of interest *in vitro* or *in vivo* in an assembly step.

2. The process according to claim 1, wherein said neutral sequence change comprises
- neutral codon replacement within protein coding sequences, and/or
- neutral base substitution, insertion, or deletion or synonymous sequence replacement within intergenic sequences.

3. The process according to claim 1 or 2, wherein a first detachable adapter sequence is added to one end of each member of each *in silico* assembly variant pool, and a second detachable adapter sequence is added to the other end of each member of each *in silico* assembly variant pool, wherein
- said first detachable adapter sequence and said second detachable adapter sequence have different sequences, and wherein optionally a first primer capable of annealing to said first detachable adapter sequence and a second primer capable of annealing to said second detachable adapter sequence are used in the amplification step; and
- said first detachable adapter sequence and said second detachable adapter sequence are removed from each member of said amplified library of nucleic acid assembly units before said assembly step.

4. The process according to claim 3, wherein said first detachable adapter sequence comprises a first primer binding region and a first cleavage site, and said second detachable adapter sequence comprises a second primer binding region and a second cleavage site, wherein said first cleavage site and said second cleavage site are specifically recognizable by different endonucleases.

5. The process according to any one of the preceding claims, wherein said DNA construct of interest is a linear nucleic acid molecule, a circular nucleic acid molecule such as a plasmid, or an artificial chromosome.

6. The process according to any one of the previous claims, said DNA construct of interest has a length of at least 10,000 base pairs, particularly of at least 1000,000 base pairs.

7. The process according to any one of the preceding claims, wherein each member of said plurality of original *in silico* assembly units independently of each other has a length in range of 500 base pairs to 3,000 base pairs.

8. The process according to any one of the preceding claims, wherein each of said terminal homology regions independently from each other has a length of 15 base pairs to 35 base pairs or above.

9. The process according to any one of the preceding claims, wherein said genetic element is select from an operon, a promoter, an open reading frame, an enhancer, a silencer, an exon, an intron, or a gene.

10. The process according to any one of the preceding claims, wherein said terminal homology region is comprised within a protein coding sequence or an intergenic sequence.

11. The process according to any one of the preceding claims, wherein said partitioning step comprises
- partitioning said optimized *in silico* DNA construct into a plurality of *in silico* segment assembly units, wherein in each case two adjacent *in silico* segments assembly units share a segment terminal homology region;
- partitioning each member of said plurality of *in silico* segments into a plurality of *in silico* block assembly units, wherein in each case two adjacent block assembly units share a block terminal homology region, and
- partitioning each member of said plurality of *in silico* block assembly units into a plurality of *in silico* subblock assembly units, wherein in each case two adjacent subblock assembly units share a subblock terminal homology region, thereby yielding said plurality of original *in silico* assembly units.

12. The process according to any one of claims 2 and 11, wherein
- said first detachable adapter sequence is or comprises a segment adapter sequence, and said second detachable adapter sequence is or comprises a block adapter sequence;
- members of each *in silico* assembly variant pool corresponding to the same *in silico* segment assembly unit have the same segment adapter sequence;
- members of each *in silico* assembly variant pool corresponding to the same *in silico* block assembly unit have the same block adapter sequence,
- each segment adapter sequence differs from each other; and
- each block adapter sequence differs from each other.

13. The process according to claim 11 or 12, wherein said assembly steps comprises
- pooling and assembling members of said amplified library of nucleic acid assembly units corresponding to an *in silico* block assembly unit into a nucleic acid block assembly unit, respectively, thereby yielding a plurality of nucleic acid block assembly units;
- pooling and assembling nucleic acid block assembly units corresponding to an *in silico* segment assembly unit into a nucleic acid segment assembly unit, respectively, thereby yielding a plurality of nucleic acid segment assembly units; and
- pooling and assembling said nucleic acid segments assembly units to said DNA of interest.

14. The process according to any one of claims 11 to 13, wherein,
- each member of said plurality of *in silico* segment assembly units independently of each other has a length in the range of 10,000 base pairs to 50,000 base pairs,
- each member of said plurality of *in silico* block assembly units independently of each other has a length in range of 2,000 base pairs to 10,000 base pairs;
- each of said segment terminal homology regions has independently from each other a length in the range of 35 base pairs to 200 base pairs; and/or
- each of said block terminal homology regions has independently from each other a length in the range of 35 base pairs to 90 base pairs.

15. A process for manufacture a variant of a DNA construct of interest, comprising the steps of
- providing an original *in silico* DNA construct comprising a plurality of genetic elements;
- subjecting said original *in silico* DNA construct to a computational optimization step, wherein one or more sequences inhibiting de novo DNA synthesis are removed from said template *in silico* DNA construct by neutral sequence change, yielding an optimized original *in silico* DNA construct, provided that start codons are not removed or replaced;
- partitioning said optimized *in silico* DNA construct into a plurality of original *in silico* assembly units in a partitioning step, wherein said optimized *in silico* DNA construct is partitioned such that in each case two adjacent members of said plurality of original *in silico* assembly units share a terminal homology region, wherein one terminal homology region differs from any other;
- subjecting each member of said plurality of original *in silico* assembly units to computational mutating sequence recoding step or a computational synonymous sequence recoding step, wherein
• in said computational mutating sequence recoding step, one or more mutant *in silico* assembly units are generated for one or more members of said plurality of original *in silico* assembly units by non-neutral sequence change, provided that no terminal homology region or start codon is altered, and an *in silico* assembly mutant pool comprising said one or more mutant *in silico* assembly units is generated, thereby yielding a respective library of *in silico* mutant pools;
• in said computational synonymous sequence recoding step, one or more synonymous *in silico* assembly units are generated for each member of said plurality of original *in silico* assembly units not being subjected to said computational mutating sequence recoding step by neutral sequence change, provided that no terminal homology region or start codon is altered, and an *in silico* assembly variant pool comprising said member of said plurality of original *in silico* assembly units and said one or more synonymous *in silico* assembly units is generated, thereby yielding a respective library of *in silico* variant pools;
- de novo synthesizing one or more members of each *in silico* assembly variant pool of said library of *in silico* variant pools and one or more members of each *in silico* mutant pool of said library of in silico mutant pools, thereby yielding a library of nucleic acid assembly units;
- amplifying said library of nucleic acid assembly units in an amplification step, yielding an amplified library of nucleic acid assembly units; and
- assembling said amplified library of nucleic acid assembly units to said variant of a DNA construct of interest *in vitro* or *in vivo* in an assembly step.
